# EUROPÄISCHE PATENTSCHRIFT

(11) **EP 1 218 378 B1**
(45) Veröffentlichungstag und Bekanntmachung des Hinweises auf die Patenterteilung: **23.04.2003**
(21) Anmeldenummer: 00969439.9
(22) Anmeldetag: 06.10.2000
(51) Int. Cl.: C07D 471/04, A61K 31/435, A61P 25/00

(54) **TERT.-BUTYL-(7-METHYL-IMIDAZO[1,2-A]PYRIDIN-3-YL)-AMIN-DERIVATE**
TERT.-BUTYL-(7-METHYL-IMIDAZO[1,2-A]PYRIDINE-3-YL)-AMINE DERIVATIVES
DERIVES DE TERT.-BUTYL-(METHYL-IMIDAZO[1,2-A]PYRIDINE-3-YL)AMINE

(30) Priorität: 08.10.1999 DE 19948434
(43) Veröffentlichungstag der Anmeldung: 03.07.2002
(73) Patentinhaber: Grünenthal GmbH, 52078 Aachen (DE)
(72) Erfinder: MAUL, Corinna, 52066 Aachen (DE); SUNDERMANN, Bernd, 52066 Aachen (DE); HENNIES, Hagen-Heinrich, 52152 Simmerath (DE); SCHNEIDER, Johannes, 52223 Stolberg (DE); GERLACH, Matthias, 63636 Brachttal (DE)
(86) Internationale Anmeldenummer: EP0009791
(87) Internationale Veröffentlichungsnummer: WO01027109

(56) Entgegenhaltungen:
- EP-A- 0 266 890
- FR-A- 2 638 161
- US-A- 3 489 755
- US-A- 4 450 164
- H.BIENAYM ET AL.: "A New Heterocyclic Multicomponent Reaction for the Combinatorial Synthesis of Fused 3-Aminoimidazoles." ANGEWANDTE CHEMIE INTERNATIONAL EDITION, Bd. 37, Nr. 16, 1998, Seiten 2234-7, XP000978871
- BLACKBURN C ET AL: "Parallel Synthesis of 3-Aminoimidazo[1,2-a]pyridines and pyrazines by a New Three-Component Condensation" TETRAHEDRON LETTERS,NL,ELSEVIER SCIENCE PUBLISHERS, AMSTERDAM, Bd. 39, Nr. 22, 28. Mai 1998 (1998-05-28), Seiten 3635-3638, XP004118699 ISSN: 0040-4039
- BLACKBURN C: "A Three-Component Solid-Phase Synthesis of 3-Aminoimidazo[1,2-a]azines" TETRAHEDRON LETTERS,NL,ELSEVIER SCIENCE PUBLISHERS, AMSTERDAM, Bd. 39, Nr. 31, 30. Juli 1998 (1998-07-30), Seiten 5469-5472, XP004124091 ISSN: 0040-4039
- F.MURAD: "Discovery of Some of the Biological Effetcs of Nitric Oxide and Its Role in Cell Signalling (Nobel Lecture)" ANGEWANDTE CHEMIE, INTERNATIONAL EDITION, Bd. 38, 1999, Seiten 1156-68, XP002159759 in der Anmeldung erwähnt

## Beschreibung

Die Erfindung betrifft *tert*.-Butyl-(7-methyl-imidazo[1,2-a]pyridin-3-yl)-amin-Derivate, ein Verfahren zur ihrer Herstellung, Arzneimittel, die diese Verbindungen enthalten, die Verwendung der erfindungsgemäßen *tert*.-Butyl-(7-methyl-imidazo[1,2-a]pyridin-3-yl)-amin-Derivate zur Herstellung eines Medikaments zur NO-Synthase-Inhibierung und zur Behandlung von u.a. Migräne sowie pharmazeutische Zusammensetzungen, die *tert*.-Butyl-(7-methyl-imidazo[1,2-a]pyridin-3-yl)-amin-Derivate enthalten.

Stickstoffmonoxid (NO) reguliert zahlreiche physiologische Prozesse, unter anderem die Neurotransmission, Relaxation und Proliferation von glatter Muskulatur, die Adhäsion und Aggregation von Thrombozyten sowie die Gewebeverletzung und Entzündung. Aufgrund der Vielzahl von Signalfunktionen wird NO mit einer Reihe von Krankheiten in Verbindung gebracht (s. z.B. L. J. Ignarro, *Angew. Chem.* (1999), 111, 2002-2013 und F. Murad, *Angew. Chem. Int. Ed.* (1999), 111, 1976-1989). Eine wichtige Rolle bei der therapeutischen Beeinflussung dieser Krankheiten spielt dabei das für die physiologische Bildung von NO verantwortliche Enzym, die NO-Synthase (NOS). Bislang wurden drei verschiedene Isoformen der NO-Synthase, nämlich die beiden konstitutiven Formen nNOS und eNOS sowie die induzierbare Form iNOS, identifiziert (A. J. Hobbs, A. Higgs, S. Moncada, *Annu. Rev. Pharmacol. Toxicol.* (1999), 39, 191-220; I. C. Green, P.-E. Chabrier, *DDT* (1999), 4, 47-49; P.-E. Chabrier et al., *Cell. Mol. Life Sci.* (1999), 55, 1029-1035).

Die Hemmung der NO-Synthase eröffnet neue Therapieansätze für verschiedene Krankheiten, die mit NO in Zusammenhang stehen (A. J.

Hobbs et al., *Annu. Rev. Pharmacol. Toxicol.* (1999). 39, 191-220; I. C. Green, P.-E. Chabrier, *DDT* (1999), 4, 47-49; P.-E. Chabrier et al., *Cell. Mol. Life Sci*. (1999), 55, 1029-1035), wie beispielsweise Migräne (L. L. Thomsen, J. Olesen, *Clinical Neuroscience* (1998), 5, 28-33; L. H. Lassen et al., *The Lancet* (1997), 349, 401-402), septischer Schock, neurodegenerative Erkrankungen wie Multiple Sklerose, Morbus Parkinson, Morbus Alzheimer oder Morbus Huntington, Entzündungen, Entzündungsschmerz, cerebrale Ischämie, Diabetes, Meningitis und Arteriosklerose. Darüber hinaus kann die NOS-Inhibierung einen Effekt auf die Wundheilung, auf Tumoren und auf die Angiogenese haben sowie eine unspezifische Immunität gegen Microorganismen bewirken (A. J. Hobbs et al., *Annu. Rev. Pharmacol. Toxicol.* (1999), 39, 191-220).

Bislang bekannte Wirkstoffe, die die NO-Synthase hemmen, sind neben L-NMMA und L-NAME - d.h. Analoga des L-Arginins, aus dem in-vivo unter Beteiligung von NOS NO und Citrullin gebildet werden - u.a. S-Methyl-Lcitrullin, Aminoguanidin, S-Methylisoharnstoff, 7-Nitroindazol und 2-Mercaptoethylguanidin (A. J. Hobbs et al., *Annu. Rev. Pharmacol. Toxicol.* (1999), 39, 191-220).

Der vorliegenden Erfindung lag demgegenüber die Aufgabe zugrunde, neue wirksame NOS-Inhibitoren zur Verfügung zu stellen.

Überraschenderweise wurde gefunden, daß 3-*tert*.-Butyl-aminosubstituierte Imidazo[1,2-a]pyridine der allgemeinen Struktur I 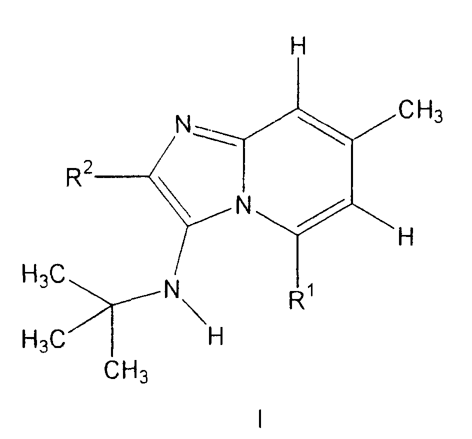 worin
- R¹: H oder C₁₋₄-Alkanyl bedeutet, wobei Alkanyl geradkettig oder verzweigt ist und unsubstituiert oder ein- oder mehrfach substituiert ist, und
- R²: C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-C₃₋₈-Cycloalkyl, C₁₋₈-Alkyl-Heterocyclyl, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Cycloalkyl gesättigt oder ungesättigt ist und unsubstituiert oder ein- oder mehrfach substituiert, Heterocyclyl gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet,
in Form einer ihrer Basen oder eines ihrer pharmazeutisch annehmbaren Salze, sehr wirksame NOS-Inhibitoren darstellen.

Diese Verbindungen sind als solche neu mit Ausnahme des 3-*tert.*-Butylamino-substituierten Imidazo[1,2-a]pyridins der allgemeinen Struktur I, worin R¹ = Methyl und R² = Phenyl bedeuten, das von H. Bienymé und K. Bouzid, *Angew. Chem.* (1998), 110, 2349-2352, beschrieben wurde, jedoch ohne eine NOS-inhibierende (oder irgendeine andere pharmakologische oder therapeutische) Wirkung zu offenbaren. Daher ist auch diese letztgenannte Verbindung Gegenstand dieser Erfindung, soweit ihre Verwendung in einem Arzneimittel und insbesondere zur Herstellung eines Medikaments zur Inhibierung von NO-Synthase und zur Behandlung von Migräne, septischem Schock, Multipler Sklerose, Morbus Parkinson, Morbus Alzheimer, Morbus Huntington, Entzündungen, Entzündungsschmerz, cerebraler Ischämie, Diabetes, Meningitis, Arteriosklerose und/oder für die Wundheilung sowie eine sie enthaltende pharmazeutische Zusammensetzung betroffen sind.

Der Ausdruck "C₁₋₈-Alkyl" umfaßt im Sinne dieser Erfindung acyclische gesättigte oder ungesättigte Kohlenwasserstoffreste, die verzweigt- oder geradkettig sowie unsubstituiert oder ein- oder mehrfach substituiert sein können, mit 1 bis 8 C-Atome, d.h. C₁₋₈-Alkanyle, C₂₋₈-Alkenyle und C₂₋₈-Alkinyle. Dabei weisen Alkenyle mindestens eine C-C-Doppelbindung und Alkinyle mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, iso-Pentyl, neo-Pentyl, n-Hexyl, 2-Hexyl, n-Octyl; Ethylenyl (Vinyl), Ethinyl, Propenyl (-CH₂CH=CH₂, -CH=CH-CH₃, -C(=CH₂)-CH₃), Propinyl (-CH-C≡CH, -C≡C-CH₃), Butenyl, Butinyl, Pentenyl, Pentinyl, Hexenyl, Hexinyl, Octenyl und Octinyl umfaßt.

Der Ausdruck "C₁₋₄-Alkanyle" umfaßt im Zusammenhang mit der vorliegenden Erfindung gesättigte acyclische Kohlenwasserstoffreste mit 1 bis 4 Kohlenstoffatomen, wobei die Reste geradkettig oder verzweigt sind sowie unsubstituiert oder ein- oder mehrfach substituiert. Vorteilhaft ist C₁₋₄-Alkanyl Methyl, Ethyl, n-Propyl, 2-Propyl, n-Butyl, iso-Butyl, sec.-Butyl oder tert.-Butyl. Besonders bervorzugt steht C₁₋₄-Alkanyl für Methyl.

Der Ausdruck "C₃₋₈-Cycloalkyl" bedeutet für die Zwecke dieser Erfindung cyclische Kohlenwasserstoffe mit 3 bis 8 Kohlenstoffatomen, die gesättigt oder ungesättigt, unsubstituiert oder ein- oder mehrfach substituiert sein können. Vorteilhaft ist C₃₋₈-Cycloalkyl aus der Gruppe ausgewählt, die Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl enthält. Besonders bevorzugt steht Cycloalkyl für Cyclohexyl.

Der Ausdruck "Heterocyclyl" steht für einen 3-, 4-, 5-, 6- oder 7-gliedrigen cyclischen organischen Rest, der mindestens 1, ggf. auch 2, 3, 4 oder 5 Heteroatome enthält, wobei die Heteroatome gleich oder verschieden sind und der cyclische Rest gesättigt oder ungesättigt, aber nicht aromatisch ist und unsubstituiert oder ein- oder mehrfach substituiert sein kann. Der Heterocyclus kann auch Teil eines bi- oder polycyclischen Systems sein. Bevorzugte Heteroatome sind Stickstoff, Sauerstoff und Schwefel. Es ist bevorzugt, daß der Heterocyclyl-Rest ausgewählt ist aus der Gruppe, die Tetrahydrofuryl, Tetrahydropyranyl, Pyrrolidinyl, Piperidinyl, Piperazinyl und Morpholinyl enthält, wobei die Bindung an die Verbindung der allgemeinen Struktur I (bzw. III) über jedes beliebige Ringglied des Heterocyclyl-Restes erfolgen kann.

Der Ausdruck "Aryl" bedeutet im Sinne dieser Erfindung aromatische Kohlenwasserstoffe, u.a. Phenyle, Naphthyle und Anthracenyle. Die Aryl-Reste können auch mit weiteren gesättigten, (partiell) ungesättigten oder aromatischen Ringsystemen kondensiert sein. Jeder Aryl-Rest kann unsubstituiert oder einfach oder mehrfach substituiert vorliegen, wobei die Aryl-Substituenten gleich oder verschieden und in jeder beliebigen und möglichen Position des Aryls sein können. Vorteilhafterweise ist Aryl aus der Gruppe ausgewählt, die Phenyl, 1-Naphthyl und 2-Naphthyl enthält. Besonders bevorzugte Aryl-Reste sind für die Zwecke der Erfindung Phenyl, 3-Hydroxyphenyl, 3-Methoxyphenyl, 2,3-Dihydroxyphenyl, 2,3-Dimethoxyphenyl und 1-Naphthyl.

Der Ausdruck "Heteroaryl" steht für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sind und der Heterocyclus unsubstituiert oder ein- oder mehrfach substituiert sein kann; im Falle der Substitution am Heterocyclus können die Heteroarylsubstituenten gleich oder verschieden sein und in jeder beliebigen und möglichen Position des Heteroaryls sein. Der Heterocyclus kann auch Teil eines bi- oder polycyclischen Systems sein. Bevorzugte Heteroatome sind Stickstoff, Sauerstoff und Schwefel. Es ist bevorzugt, daß der Heteroaryl-Rest ausgewählt ist aus der Gruppe, die Pyrrolyl, Furyl, Thienyl, Pyrazolyl, Imidazolyl, Pyridinyl, Pyridazinyl, Pyrimidinyl, Pyrazinyl, Pyranyl, Indolyl, Indazolyl, Purinyl, Pyrimidinyl, Indolizinyl, Chinolinyl, Isochinolinyl, Chinazolinyl, Carbazolyl, Phenazinyl, Phenothiazinyl enthält, wobei die Bindung an die Verbindungen der allgemeinen Struktur I (bzw. III) über jedes beliebige und mögliche Ringglied des Heteroaryl-Restes erfolgen kann. Besonders bevorzugte Heteroaryl-Reste sind für die Zwecke dieser Erfindung Pyridin-2-yl, Pyridin-3-yl, Furan-2-yl, Furan-3-yl, 5-Hydroxymethylen-furan-2-yl, 5-Nitro-furan-2-yl, 5-[1,3]-dioxolan-furan-2-yl, 5-Carbonsäure-furan-2-yl, Thien-2-yl (2-Thiophen), Thien-3-yl (3-Thiophen) und 5-Carbonsäure-2-thiophen (5-Carbonsäure-thien-2-yl).

Die Ausdrücke "C₁₋₈-Alkyl-C₃₋₈-Cycloalkyl", "C₁₋₈-Alkyl-Heterocyclyl", "C₁₋₈-Alkyl-Aryl" oder "C₁₋₈-Alkyl-Heteroaryl" bedeuten für die Zwecke der vorliegenden Erfindung, daß C₁₋₈-Alkyl und Cycloalkyl. Heterocyclyl, Aryl und Heteroaryl die oben definierten Bedeutungen haben und der Cycloalkyl-, Heterocyclyl-, Aryl- bzw. Heteroaryl-Rest über eine C₁₋₈-Alkyl-Gruppe an die Verbindung der allgemeinen Struktur I (bzw. III) gebunden ist.

Im Zusammenhang mit "Alkyl", "Alkanyl", "Alkenyl" und "Alkinyl" versteht man unter dem Begriff "substituiert" im Sinne dieser Erfindung die Substitution eines Wasserstoffrestes durch F, Cl, Br, I, CN, NH₂, NH-Alkyl, NH-Aryl, NH-Heteroaryl, NH-Alkyl-Aryl, NH-Alkyl-Heteroaryl, NH-Heterocyclyl, NH-Alkyl-OH, N(Alkyl)₂, N(Alkyl-Aryl)₂, N(Alkyl-Heteroaryl)₂, N(Heterocyclyl)₂, N(Alkyl-OH)₂, NO, NO₂, SH, S-Alkyl, S-Aryl, S-Heteroaryl, S-Alkyl-Aryl, S-Alkyl-Heteroaryl, S-Heterocyclyl, S-Alkyl-OH, S-Alkyl-SH, OH, O-Alkyl, O-Aryl, O-Heteroaryl, O-Alkyl-Aryl, O-Alkyl-Heteroaryl, O-Heterocyclyl, O-Alkyl-OH, CHO, C(=O)C₁₋₆-Alkyl, C(=S)C₁₋₆-Alkyl, C(=O)Aryl, C(=S)Aryl, C(=O)C₁₋₆-Alkyl-Aryl, 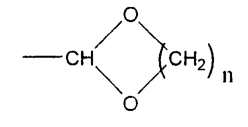 mit n = 1, 2 oder 3, C(=S)C₁₋₆-Alkyl-Aryl, C(=O)-Heteroaryl C(=S)-Heteroaryl, C(=O)-Heterocyclyl, C(=S)-Heterocyclyl, CO₂H, CO₂-Alkyl, CO₂-Alkyl-Aryl, C(=O)NH₂, C(=O)NH-Alkyl, C(=O)NHAryl, C(=O)NH-Heterocyclyl, C(=O)N(Alkyl)₂, C(=O)N(Alkyl-Aryl)₂, C(=O)N(Alkyl-Heteroaryl)₂, C(=O)N(Heterocyclyl)₂, SO-Alkyl, SO₂-Alkyl, SO₂NH₂, SO₃H, Cycloalkyl, Aryl, Heteroaryl oder Heterocyclyl. wobei unter mehrfach substituierten Resten solche Reste zu verstehen sind, die entweder an verschiedenen oder an gleichen Atomen mehrfach. z.B. zwei- oder dreifach, substituiert sind, beispielsweise dreifach am gleichen C-Atom wie im Falle von CF₃ oder -CH₂CF₃ oder an verschiedenen Stellen wie im Falle von -CH(OH)-CH=CH-CHCl₂. Die Mehrfachsubstitution kann mit dem gleichen oder mit verschiedenen Substituenten erfolgen. Besonders bevorzugt für die Zwecke der vorliegenden Erfindung bedeutet "Alkyl" in diesem Zusammenhang Methyl, Ethyl, CH₂-OH oder CF₃.

In Bezug auf "Aryl", "Heterocyclyl", "Heteroaryl" und "Alkyl-Aryl" sowie "Cycloalkyl" versteht man im Sinne dieser Erfindung unter "ein- oder mehrfach substituiert" die ein- oder mehrfache, z.B. zwei-, drei- oder vierfache. Substitution eines oder mehrerer Wasserstoffatome des Ringsystems durch F, Cl, Br, I, CN, NH₂, NH-Alkyl, NH-Aryl, NH-Heteroaryl, NH-Alkyl-Aryl, NH-Alkyl-Heteroaryl, NH-Heterocyclyl, NH-Alkyl-OH, N(Alkyl)₂, N(Alkyl-Aryl)₂, N(Alkyl-Heteroaryl)₂, N(Heterocyclyl)₂, N(Alkyl-OH)₂, NO, NO₂, SH, S-Alkyl, S-Cycloalkyl, S-Aryl, S-Heteroaryl, S-Alkyl-Aryl, S-Alkyl-Heteroaryl, S-Heterocyclyl, S-Alkyl-OH, S-Alkyl-SH, OH, O-Alkyl, O-Cycloalkyl, O-Aryl, O-Heteroaryl, O-Alkyl-Aryl, O-Alkyl-Heteroaryl, O-Heterocyclyl, O-Alkyl-OH, CHO, C(=O)C₁₋₆-Alkyl, C(=S)C₁₋₆-Alkyl, C(=O)Aryl, C(=S)Aryl, C(=O)-C₁₋₆-Alkyl-Aryl, 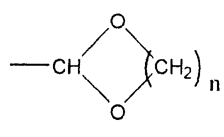 mit n = 1, 2 oder 3, C(=S)C₁₋₆-Alkyl-Aryl, C(=O)-Heteroaryl, C(=S)-Heteroaryl, C(=O)-Heterocyclyl, C(=S)-Heterocyclyl, CO₂H, CO₂-Alkyl, CO₂-Alkyl-Aryl, C(=O)NH₂, C(=O)NH-Alkyl, C(=O)NHAryl, C(=O)NH-Heterocyclyl, C(=O)N(Alkyl)₂, C(=O)N(Alkyl-Aryl)₂, C(=O)N(Alkyl-Heteroaryl)₂, C(=O)N(Heterocyclyl)₂, S(O)-Alkyl, S(O)-Aryl, SO₂-Alkyl, SO₂-Aryl, SO₂NH₂, SO₃H, CF₃, =O, =S; Alkyl, Cycloalkyl, Aryl, Heteroaryl und/oder Heterocyclyl; an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten. Für "Aryl" sind dabei besonders bevorzugte Substituenten -F, -CF₃, -OH und -O-CH₃. Für "Heteroaryl" sind besonders bevorzugte Substituenten -OH, -O-CH₃, -CH₂OH, -NO₂, -CO₂H, -CO₂Ethyl und -[1,3]-Dioxolan. Für "Cycloalkyl" sind besonders bevorzugte Substituenten CO₂H und CO₂Ethyl.

Pharmazeutisch annehmbare Salze im Sinne dieser Erfindung sind solche Salze der erfindungsgemäßen Verbindungen gemäß der allgemeinen Struktur I, die bei pharmazeutischer Verwendung physiologisch-insbesondere bei Anwendung am Säugetier und/oder Menschen-verträglich sind. Solche pharmazeutisch annehmbaren Salze können beispielsweise mit anorganischen oder organischen Säuren gebildet werden.

Vorzugsweise werden die pharmazeutisch annehmbaren Salze der erfindungsgemäßen Verbindungen gemäß der allgemeinen Struktur I mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure gebildet. Bei den gebildeten Salzen handelt es sich u.a. um Hydrochloride, Hydrobromide, Phosphate, Carbonate, Hydrogencarbonate, Formiate, Acetate, Oxalate, Succinate, Tartrate, Fumarate, Citrate und Glutaminate. Ebenfalls bevorzugt sind Solvate und insbesondere die Hydrate der erfindungsgemäßen Verbindungen, die z.B. durch Kristallisation aus wäßriger Lösung erhalten werden können.

Sofern die erfindungsgemäßen Verbindungen der allgemeinen Struktur I mindestens ein Asymmetriezentrum aufweisen, können sie in Form ihrer Racemate, in Form der reinen Enantiomeren und/oder Diastereomeren oder in Form von Mischungen dieser Enantiomeren bzw. Diastereomeren vorliegen, und zwar sowohl in Substanz als auch als pharmazeutisch annehmbare Salze dieser Verbindungen. Die Mischungen können in jedem beliebigen Mischungsverhältnis der Stereoisomeren vorliegen. Chirale Verbindungen der allgemeinen Struktur I liegen bevorzugt als enantiomerenreine Verbindungen vor.

Bevorzugte Verbindungen der vorliegenden Erfindung sind solche 3-*tert*.-Butyl-amino-substituierte lmidazo[1,2-a]pyridine der allgemeinen Struktur I, in denen R¹ H oder Methyl bedeutet, und zwar sowohl in Form ihrer Basen als auch in Form annehmbarer pharmazeutischer Salze.

Unter diesen bevorzugten Verbindungen besonders bevorzugt sind solche, in denen R² für Aryl oder Heteroaryl, insbesondere für Phenyl, 1-Naphthyl, Furyl, Thienyl oder Pyridinyl steht. Diese Reste sind dabei ganz besonders bevorzugt unsubstituiert oder mit -F, -CF₃, -OH, -OCH₃, -CH₂OH, -NO₂,-CO₂H oder -[1,3]-Dioxolan ein- oder zweifach substituiert, wobei im Falle der Zweifachsubstitution die Substitution mit verschiedenen oder mit dem gleichen Substituenten erfolgen kann. Auch diese erfindungsgemäßen Verbindungen können in Form ihrer Basen oder ihrer pharmazeutisch annehmbaren Salze vorliegen.

Die am meisten bevorzugten Verbindungen der vorliegenden sind Substanzen der allgemeinen Struktur I in Form ihrer Basen oder ihrer pharmazeutisch annehmbaren Salze, die aus der folgenden Gruppe ausgewählt sind:
*tert.*-Butyl-(7-methyl-2-pyridin-3-yl-imidazo[1,2-a]pyridin-3-yl)-amin,
*tert*.-Butyl-[2-(2,3-dimethoxy-phenyl)-5,7-dimethyl-imidazo[1,2-a]pyridin-3-yl]-amin,
3-(3*-tert.*-Butylamino-5,7-dimethyl-imidazo[1,2-a]pyridin-2-yl)-phenol 3-(3-*tert*.-Butylamino-7-methyl-imidazo[1,2-a]pyridin-2-yl)-phenol,
*tert*.-Butyl-(2-furan-2-yl-5,7-dimethyl-imidazo[1,2-a]pyridin-3-yl)-amin,
*tert.*-Butyl-(7-methyl-2-naphthalen-1-yl-imidazo[1,2-a]pyridin-3-yl )-amin,
*tert*.-Butyl-[5,7-dimethyl-2-(5-nitro-furan-2-yl)-imidazo[1,2-a]pyridin-3-yl]-amin,
[5-(3-*tert*.-Butylamino-7-methyl-imidazo[1,2-a]pyridin-2-yl)-furan-2-yl]-methanol,
*tert*.-Butyl-[2-(5-[1,3]dioxolan-2-yl-furan-2-yl)-7-methyl-imidazo[1,2-a]pyridin-3-yl]-amin,
5-(3-*tert*.-Butylamino-57-dimethyl-imidazo[1,2-a]pyridin-2-yl)-furan-2-carbonsäure,
*tert*.-Butyl-(2-furan-2-yl-7-methyl-imidazo[1,2-a]pyridin-3-yl)-amin,
*tert*.-Butyl-(7-methyl-2-pyridin-2-yl-imidazo[1,2-a]pyridin-3-yl)-amin,
5-(3-*tert*.-Butylamino-7-methyl-imidazo[1,2-a]pyridin-2-yl)-thiophen-2-carbonsäure,
*tert*.-Butyl-(7-methyl-2-phenyl-imidazo[1,2-a]pyridin-3-yl)-amin.

Weitere bevorzugte Verbindungen der allgemeinen Struktur I in Form ihrer Basen bzw. ihrer pharmazeutisch annehmbaren Salze sind aus der folgenden Gruppe ausgewählt:
*tert*.-Butyl-(2,5,7-trimethyl-imidazo[1,2-a]pyridin-3-yl)-amin,
*tert*.-Butyl-(2-cyclohexyl-5,7-dimethyl-imidazo[1,2-a]pyridin-3-yl)-amin,
*tert*.-Butyl-(2-cyclohexyl-7-methyl-imidazo[1,2-a]pyridin-3-yl)-amin,
*tert*.-Butyl-(2,7-dimethyl-imidazo[1,2-a]pyridin-3-yl)-amin.

Die vorliegende Erfindung betrifft auch ein Verfahren zur Herstellung einer Verbindung der allgemeinen Struktur I 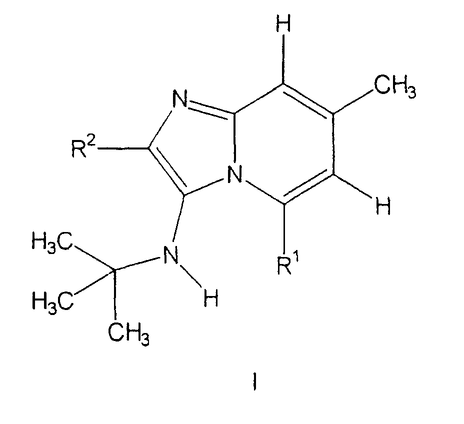 wobei dieses Verfahren dadurch gekennzeichnet ist, daß ein Aminopyridin der allgemeinen Struktur II 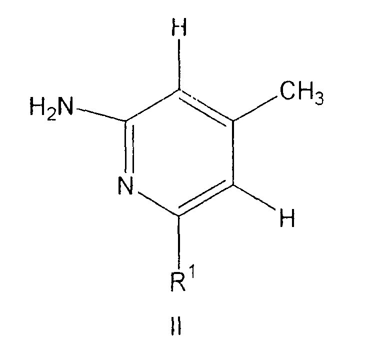 worin
- R¹: H oder C₁₋₄-Alkanyl bedeutet, wobei Alkanyl geradkettig oder verzweigt ist und unsubstituiert oder ein- oder mehrfach substituiert ist,
mit einem Aldehyd der allgemeinen Struktur III 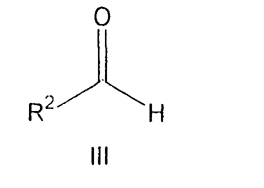 worin
- R²: C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-C₃₋₈-Cycloalkyl, C₁₋₈-Alkyl-Heterocyclyl, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Cycloalkyl gesättigt oder ungesättigt ist und unsubstituiert oder ein- oder mehrfach substituiert, Heterocyclyl gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet,

unter der Maßgabe, daß R² nicht Phenyl bedeutet, wenn R¹ Methyl bedeutet.
und *tert*.-Butyl-isonitril der Struktur IV 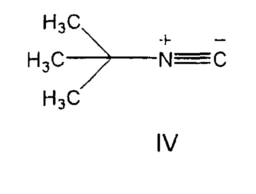 umgesetzt wird.

Es ist bevorzugt, das erfindungsgemäße Verfahren in Gegenwart einer anorganischen oder organischen Lewis- oder Protonen-Säure, insbesondere in Gegenwart von Perchlorsäure, die vorzugsweise als 20 %ige wäßrige Lösung eingesetzt wird, durchzuführen.

Bevorzugt wird die erfindungsgemäße Dreikomponentenreaktion in einem "Eintopf"-Verfahren durchgeführt, wobei je ein Aminopyridin der allgemeinen Struktur II mit je einem Aldehyd der allgemeinen Struktur III und dem Isonitril IV gleichzeitig miteinander zur Reaktion gebracht wird.

Das erfindungsgemäße Verfahren kann auch in semi- oder vollautomatisierter Form als Parallelsynthese einer Gruppe von erfindungsgemäßen Verbindungen der allgemeinen Struktur I durchgeführt werden.

Das erfindungsgemäße Verfahren kann lösungsmittelfrei durchgeführt werden. Vorzugsweise wird das Verfahren in einem organischen Lösungsmittel, insbesondere Dichlormethan oder Acetonitril, durchgeführt. Reaktionstemperatur und Reaktionszeit werden dabei vorzugsweise so gewählt, daß eine möglichst vollständige Umsetzung der Edukte erreicht wird. Bevorzugt liegt die Umsetzungstemperatur bei 0 °C bis 80 °C, insbesondere bei 10 °C bis 35 °C. Die Reaktionszeit liegt üblicherweise zwischen 5 min und 24 h.

Die im erfindungsgemäßen Verfahren eingesetzten Aminopyridine der allgemeinen Struktur II, die Aldehyde der allgemeinen Struktur III und das *tert*.-Butyl-isonitril der Struktur IV sind kommerziell erhältlich (von Acros, Geel; Avocado, Port of Heysham; Aldrich, Deisenhofen; Fluka, Seelze; Lancaster, Mülheim; Maybridge, Tintagel; Merck, Darmstadt; Sigma, Deisenhofen; TCI, Japan) oder können nach im Stand der Technik allgemein bekannten Verfahren hergestellt werden.

Die erfindungsgemäßen Verbindungen der allgemeinen Struktur I können sowohl als freie Base als auch als Salz isoliert werden. Die freie Base der erfindungsgemäßen Verbindung der allgemeinen Struktur I wird üblicherweise nach erfolgter Umsetzung gemäß dem oben beschriebenen erfindungsgemäßen Verfahren und anschließender herkömmlicher Aufarbeitung erhalten. Die so gewonnene oder in-situ ohne Isolierung gebildete freie Base kann dann beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, in das korrespondierende Salz überführt werden. Bei den gebildeten Salzen handelt es sich u.a. um Hydrochloride, Hydrobromide, Phosphate, Carbonate, Hydrogencarbonate, Formiate, Acetate, Oxalate, Succinate, Tartrate, Fumarate, Citrate und Glutaminate. Die besonders bevorzugte Hydrochloridbildung kann auch durch Versetzen der in einem geeigneten organischen Lösungsmittel, wie z.B. Butan-2-on (Methylethylketon), gelösten Base mit Trimethylsilylchlorid (TMSCI) herbeigeführt werden.

Soweit die Verbindungen der allgemeinen Struktur I in dem erfindungsgemäßen Herstellungsverfahren als Racemate oder als Mischungen ihrer verschiedenen Enantiomeren und/oder Diastereomeren erhalten werden, können diese Mischungen nach im Stand der Technik wohlbekannten Verfahren aufgetrennt werden. Geeignete Methoden sind u.a. chromatographische Trennverfahren, insbesondere Flüssigkeitschromatographie-Verfahren unter Normal- oder erhöhtem Druck, bevorzugt MPLC- und HPLC-Verfahren, sowie Verfahren der fraktionierten Kristallisation. Dabei können insbesondere einzelne Enantiomeren z.B. mittels HPLC an chiraler Phase oder mittels Kristallisation von mit chiralen Säuren, etwa (+)-Weinsäure, (-)-Weinsäure oder (+)-10-Camphersulfonsäure, gebildeten diastereomeren Salzen voneinander getrennt werden.

Weiterhin ist ein Arzneimittel, das mindestens eine Verbindung der allgemeinen Struktur I in Form ihrer Base oder eines pharmazeutisch annehmbaren Salzes enthält 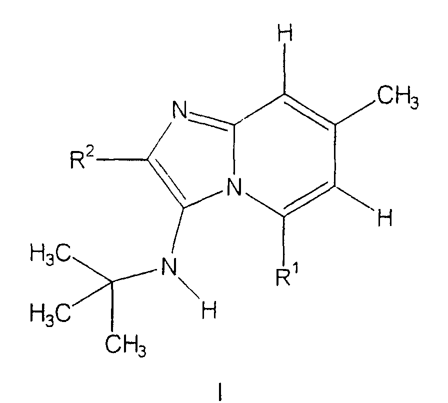 worin
- R¹: H oder C₁₋₄-Alkanyl bedeutet, wobei Alkanyl geradkettig oder verzweigt ist und unsubstituiert oder ein- oder mehrfach substituiert ist, und
- R²: C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-C₃₋₈-Cycloalkyl, C₁₋₈-Alkyl-Heterocyclyl, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Cycloalkyl gesättigt oder ungesättigt ist und unsubstituiert oder ein- oder mehrfach substituiert, Heterocyclyl gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet,

Gegenstand der Erfindung. Bevorzugt ist dabei, daß das Arzneimittel die erfindungsgemäße Verbindung der allgemeinen Struktur I in Form ihres Hydrochloridsalzes enthält. Besonders bevorzugt enthält das erfindungsgemäße Arzneimittel eine Verbindung in Form ihrer Base oder eines pharmazeutisch annehmbaren Salzes, insbesondere in Form ihres Hydrochlorids, die ausgewählt ist aus:
*tert*.-Butyl-(7-methyl-2-pyridin-3-yl-imidazo[1,2-a]pyridin-3-yl)-amin,
*tert*.-Butyl-[2-(2,3-dimethoxy-phenyl)-5,7-dimethyl-imidazo[1,2-a]pyridin-3-yl]-amin,
*tert*.-Butyl-(2,5,7-trimethyl-imidazo[1,2-a]pyridin-3-yl)-amin,
3-(3-*tert*.-Butylamino-57-dimethyl-imidazo[12-a]pyridin-2-yl)-phenol
*tert*.-Butyl-(2-cyclohexyl-5,7-dimethyl-imidazo[1,2-a]pyridin-3-yl)-amin,
3-(3-*tert*.-Butylamino-7-methyl-imidazo[1,2-a]pyridin-2-yl)-phenol,
*tert*.-Butyl-(2-furan-2-yl-5,7-dimethyl-imidazo[1,2-a]pyridin-3-yl)-amin,
*tert*.-Butyl-(7-methyl-2-naphthalen-1-yl-imidazo[1,2-a]pyridin-3-yl)-amin, *tert*.-Butyl-[5,7-dimethyl-2-(5-nitro-furan-2-yl)-imidazo[1,2-a]pyridin-3-yl]-amin,
[5-(3-*tert*.-Butylamino-7-methyl-imidazo[1,2-a]pyridin-2-yl)-furan-2-yl]-methanol,
*tert*.-Butyl-[2-(5-[1,3]dioxolan-2-yl-furan-2-yl)-7-methyl-imidazo[1,2-a]pyridin-3-yl]-amin,
5-(3-*tert*.-Butylamino-5,7-dimethyl-imidazo[1,2-a]pyridin-2-yl)-furan-2-carbonsäure,
*tert*.-Butyl-(2-furan-2-yl-7-methyl-imidazo[1,2-a]pyridin-3-yl)-amin,
*tert*.-Butyl-(7-methyl-2-pyridin-2-yl-imidazo[1,2-a]pyridin-3-yl)-amin,
*tert*.-Butyl-(2-cyclohexyl-7-methyl-imidazo[1,2-a]pyridin-3-yl)-amin,
*tert*.-Butyl-(2,7-dimethyl-imidazo[1,2-a]pyridin-3-yl)-amin,
5-(3-*tert*.-Butylamino-7-methyl-imidazo[1,2-a]pyridin-2-yl)-thiophen-2-carbonsäure,
*tert.*-Butyl-(7-methyl-2-phenyl-imidazo[1,2-a]pyridin-3-yl)-amin.

Ein weiterer Gegenstand der Erfindung ist die Verwendung einer erfindungsgemäßen Verbindung der allgemeinen Struktur I 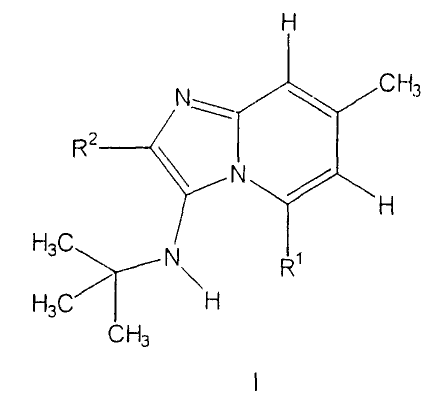 worin
- R¹: H oder C₁₋₄-Alkanyl bedeutet, wobei Alkanyl geradkettig oder verzweigt ist und unsubstituiert oder ein- oder mehrfach substituiert ist, und
- R²: C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-C₃₋₈-Cycloalkyl, C₁₋₈-Alkyl-Heterocyclyl, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Cycloalkyl gesättigt oder ungesättigt ist und unsubstituiert oder ein- oder mehrfach substituiert, Heterocyclyl gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet.
in Form ihrer Base oder eines pharmazeutisch annehmbaren Salzes, einschließlich möglicher Stereoisomeren oder racemischer oder nichtracemischer Stereoisomerengemische, insbesondere in Form des Hydrochloridsalzes, zur Herstellung eines Medikaments zur NO-Synthase-Inhibierung. Die erfindungsgemäßen Verbindungen der allgemeinen Struktur I haben sich überraschenderweise als wirksame NOS-Inhibitoren erwiesen.

Besonders bevorzugt für die erfindungsgemäße Verwendung ist eine Verbindung der allgemeinen Struktur I in Form ihrer Base oder eines pharmazeutisch annehmbaren Salzes, die ausgewählt ist aus:
*tert*.-Butyl-(7-methyl-2-pyridin-3-yl-imidazo[1,2-a]pyridin-3-yl)-amin,
*tert*.-Butyl-[2-(2,3-dimethoxy-phenyl)-57-dimethyl-imidazo[1,2-a]pyridin-3-yl]-amin,
*tert*.-Butyl-(2,5,7-trimethyl-imidazo[1,2-a]pyridin-3-yl)-amin,
3-(3-*tert*.-Butylamino-5,7-dimethyl-imidazo[1,2-a]pyridin-2-yl)-phenol
*tert*.-Butyl-(2-cyclohexyl-5,7-dimethyl-imidazo[1,2-a]pyridin-3-yl)-amin,
3-(3-*tert*.-Butylamino-7-methyl-imidazo[1,2-a]pyridin-2-yl)-phenol,
*tert*.-Butyl-(2-furan-2-yl-5,7-dimethyl-imidazo[1,2-a]pyridin-3-yl)-amin,
*tert*.-Butyl-(7-methyl-2-naphthalen-1-yl-imidazo[1,2-a]pyridin-3-yl)-amin,
*tert*.-Butyl-[5,7-dimethyl-2-(5-nitro-furan-2-yl)-imidazo[1,2-a]pyridin-3-yl]-amin,
[5-(3-*tert*.-Butylamino-7-methyl-imidazo[1,2-a]pyridin-2-yl)-furan-2-yl]-methanol,
*tert*.-Butyl-[2-(5-[1,3]dioxolan-2-yl-furan-2-yl)-7-methyl-imidazo[1,2-a]pyridin-3-yl]-amin,
5-(3-*tert*.-Butylamino-5,7-dimethyl-imidazo[1,2-a]pyridin-2-yl)-furan-2-carbonsäure,
*tert*.-Butyl-(2-furan-2-yl-7-methyl-imidazo[1,2-a]pyridin-3-yl)-amin,
*tert*.-Butyl-(7-methyl-2-pyridin-2-yl-imidazo[1,2-a]pyridin-3-yl)-amin,
*tert*.-Butyl-(2-cyclohexyl-7-methyl-imidazo[1,2-a]pyridin-3-yl)-amin,
*tert*.-Butyl-(2,7-dimethyl-imidazo[1,2-a]pyridin-3-yl)-amin
5-(3-*tert*.-Butylamino-7-methyl-imidazo[1,2-a]pyridin-2-yl)-thiophen-2-carbonsäure,
*tert.*-Butyl-(7-methyl-2-phenyl-imidazo[1,2-a]pyridin-3-yl)-amin.

Die erfindungsgemäßen 3-*tert*.-Butyl-amino-substituierten Imidazo[1,2-a]pyridine der allgemeinen Struktur I können in Form ihrer freien Base oder eines ihrer pharmazeutisch annehmbaren Salze insbesondere zur Herstellung eines Medikaments zur Behandlung von Migräne verwendet werden. Besonders bevorzugt sind die Verbindungen dabei ausgewählt aus der Gruppe, die enthält:
*tert*.-Butyl-(7-methyl-2-pyridin-3-yl-imidazo[1,2-a]pyridin-3-yl)-amin,
*tert*.-Butyl-[2-(2,3-dimethoxy-phenyl)-5,7-dimethyl-imidazo[1,2-a]pyridin-3-yl]-amin,
*tert*.-Butyl-(2,5,7-trimethyl-imidazo[1,2-a]pyridin-3-yl)-amin,
3-(3-*tert*.-Butylamino-5,7-dimethyl-imidazo[1,2-a]pyridin-2-yl)-phenol
*tert*.-Butyl-(2-cyclohexyl-5,7-dimethyl-imidazo[1,2-a]pyridin-3-yl)-amin,
3-(3*-tert*.-Butylamino-7-methyl-imidazo[1,2-a]pyridin-2-yl)-phenol,
*tert*.-Butyl-(2-furan-2-yl-5,7-dimethyl-imidazo[1,2-a]pyridin-3-yl)-amin,
*tert*.-Butyl-(7-methyl-2-naphthalen-1-yl-imidazo[1,2-a]pyridin-3-yl)-amin, *tert*.-Butyl-[5,7-dimethyl-2-(5-nitro-furan-2-yl)-imidazo[1,2-a]pyridin-3-yl]-amin,
[5-(3-*tert*.-Butylamino-7-methyl-imidazo[1,2-a]pyridin-2-yl)-furan-2-yl]-methanol,
*tert*.-Butyl-[2-(5-[1,3]dioxolan-2-yl-furan-2-yl)-7-methyl-imidazo[1,2-a]pyridin-3-yl]-amin,
5-(3-*tert*.-Butylamino-5,7-dimethyl-imidazo[1,2-a]pyridin-2-yl)-furan-2-carbonsäure,
*tert*.-Butyl-(2-furan-2-yl-7-methyl-imidazo[1,2-a]pyridin-3-yl)-amin,
*tert*.-Butyl-(7-methyl-2-pyridin-2-yl-imidazo[1,2-a]pyridin-3-yl)-amin,
*tert*.-Butyl-(2-cyclohexyl-7-methyl-imidazo[1,2-a]pyridin-3-yl)-amin,
*tert*.-Butyl-(2,7-dimethyl-imidazo[12-a]pyridin-3-yl)-amin,
5-(3-*tert*.-Butylamino-7-methyl-imidazo[12-a]pyridin-2-yl)-thiophen-2-carbonsäure,
*tert*.-Butyl-(7-methyl-2-phenyl-imidazo[1,2-a]pyridin-3-yl)-amin.

Darüber hinaus eignen sich die erfindungsgemäßen Verbindungen der allgemeinen Struktur I auch zur Herstellung von Medikamenten zur Behandlung von septischem Schock, Multipler Sklerose, Morbus Parkinson, Morbus Alzheimer, Morbus Huntington, Entzündungen, Entzündungsschmerz, cerebraler Ischämie, Diabetes, Meningitis, Arteriosklerose und/oder für die Wundheilung.

Ferner sind pharmazeutische Zusammensetzungen Gegenstand der vorliegenden Erfindung, die mindestens eine Verbindung der wie oben definierten allgemeinen Struktur I in Form ihrer Base oder eines ihrer pharmazeutisch annehmbaren Salze und einen oder mehrere pharmazeutische Hilfsstoffe enthalten.

Die erfindungsgemäßen Arzneimittel und pharmazeutischen Zusammensetzungen können als flüssige, halbfeste oder feste Arzneiformen und in Form von z.B. Injektionslösungen, Tropfen, Säften, Sirupen, Sprays, Suspensionen, Granulaten, Tabletten, Pellets, Patches, Kapseln, Pflastern, Zäpfchen, Salben, Cremes, Lotionen, Gelen, Emulsionen oder Aerosolen vorliegen und verabreicht werden und enthalten neben mindestens einer erfindungsgemäßen Verbindung der allgemeinen Struktur I je nach galenischer Form pharmazeutische Hilfsstoffe, wie z.B. Trägermaterialien, Füllstoffe, Lösungsmittel, Verdünnungsmittel, oberflächenaktive Stoffe, Farbstoffe, Konservierungsstoffe, Sprengmittel, Gleitmittel, Schmiermittel, Aromen und/oder Bindemittel. Diese Hilfsstoffe können beispielsweise sein: Wasser, Ethanol, 2-Propanol, Glycerin, Ethylenglycol, Propylenglycol, Polyethylenglycol, Polypropylenglycol, Glucose, Fructose, Lactose, Saccharose, Dextrose. Melasse, Stärke, modifizierte Stärke, Gelatine, Sorbitol, Inositol, Mannitol, mikrokristalline Cellulose, Methylcellulose, Carboxymethylcellulose, Celluloseacetat, Schellack, Cetylalkohol, Polyvinylpyrrolidon, Paraffine, Wachse, pharmazeutisch annehmbare natürliche und synthetische Gummis, Akaziengummi, Alginate, Dextran, gesättigte und ungesättigte Fettsäuren, Stearinsäure, Magnesiumstearat, Zinkstearat, Glycerylstearat, Natriumlaurylsulfat, genießbare Öle, Sesamöl, Kokusnußöl, Erdnußöl, Sojabohnenöl, Lecithin, Natriumlactat, Polyoxyethylen- und -propylen-fettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumdioxid, Titanoxid, Titandioxid, Magnesiumsulfat, Zinksulfat, Calciumsulfat, Pottasche, Calciumphosphat, Dicalciumphosphat, Kaliumbromid, Kaliumiodid, Talkum, Kaolin, Pectin, Crospovidon, Agar und Bentonit.

Die Auswahl der Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich u.a. Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen der allgemeinen Struktur I in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Verbindungen der allgemeinen Struktur I verzögert freisetzen.

Die Herstellung der erfindungsgemäßen Arzneimittel und pharmazeutischen Zusammensetzungen erfolgt mit Hilfe von im Stand der Technik der pharmazeutischen Formulierung wohlbekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind.

So kann z.B. für eine feste Formulierung, wie eine Tablette, der Wirkstoff des Arzneimittels, d.h. eine Verbindung der allgemeinen Struktur I oder eines ihrer pharmazeutisch annehmbaren Salze, mit einem pharmazeutischen Träger, z.B. herkömmlichen Tabletteninhaltsstoffen, wie Maisstärke, Lactose, Saccharose, Sorbitol, Talkum, Magnesiumstearat, Dicalciumphosphat oder Gummi, und pharmazeutischen Verdünnungsmitteln, wie z.B. Wasser, gemischt werden, um eine feste Präformulierungs-Zusammensetzung zu bilden, die eine erfindungsgemäße Verbindung oder ein pharmazeutisch annehmbares Salz davon in homogener Verteilung enthält. Unter einer homogenen Verteilung wird hier verstanden, daß der Wirkstoff gleichmäßig über die gesamte Präformulierungs-Zusammensetzung verteilt ist, so daß diese ohne weiteres in gleich wirksame Einheitsdosis-Formen, wie Tabletten, Pillen oder Kapseln, unterteilt werden kann. Die feste Präformulierungs-Zusammensetzung wird anschließend in Einheitsdosis-Formen unterteilt. Die Tabletten oder Pillen des erfindungsgemäßen Arzneimittels bzw. der erfindungsgemäßen Zusammensetzungen können auch beschichtet oder auf andere Weise kompoundiert werden, um eine Dosisform mit verzögerter Freisetzung bereitzustellen. Geeignete Beschichtungsmittel sind u.a. polymere Säuren und Mischungen von polymeren Säuren mit Materialien wie z.B. Schellack, Cetylalkohol und/oder Celluloseacetat.

Die an den Patienten zu verabreichende Wirkstoffmenge variiert und ist abhängig vom Gewicht, dem Alter und der Krankheitsgeschichte des Patienten, sowie von der Applikationsart, der Indikation und dem Schweregrad der Erkrankung. Üblicherweise werden 0,1 bis 5000 mg/kg, insbesondere 1 bis 500 mg/kg, vorzugsweise 2 bis 250 mg/kg Körpergewicht wenigstens einer erfindungsgemäßen Verbindung der allgemeinen Struktur I appliziert.

Nachfolgend werden die zur Bestimmung der NOS-Inhibition durch die erfindungsgemäßen Verbindungen der allgemeinen Struktur I verwendeten Assays beschrieben:

### NOS-Assay

### Allgemeines

Dieser Assay erlaubt die Bestimmung der prozentualen Hemmung von NO-Synthase durch einen Wirkstoff mittels Messung der NOS-Aktivität bei Einwirken des Wirkstoffs. Dabei wird NO-Synthase zusammen mit radioaktiv markiertem Arginin und dem Wirkstoff unter geeigneten Bedingungen gemischt. Nach Abbruch der NO-Bildungsreaktion zu einem vorgegebenen Zeitpunkt wird die Menge an nicht umgesetztem Arginin direkt oder indirekt bestimmt. Der Vergleich dieser Menge mit der in einem ohne Zusatz von Wirkstoff und unter sonst gleichen Bedingungen aus der Mischung von NOS und Arginin zurückbleibenden Menge an Arginin ergibt die %-Hemmung von NO-Synthase durch den getesteten Wirkstoff. Dieser Assay läßt sich wie folgt durchführen:
(a) Inkubation der NO-Synthase mit markiertem Arginin als Substrat in einem Reaktionsgefäß,
(b) Trennung des markierten Arginins von dem gegebenenfalls als Produkt der enzymatischen Reaktion entstandenen, markierten Citrullin zu einem Zeitpunkt, zu dem die Konzentration an Citrullin ansteigt,
(c) Messung der Menge an jeweils abgetrenntem Arginin.
Die Trennung erfolgt über eine Filterplatten-Membran.

Dieser NOS-Assay eignet sich insbesondere für ein "High Throughput Screening" (HTS) auf Mikrotiterplatten (MTP).

### HTS-NOS-Assay: Allgemeine Verfahrensweise

In diesem HTS-NOS-Assay wird radioaktives Arginin als Substrat benutzt. Das Assayvolumen kann je nach Art der Mikrotiterplatte (MTP) im Bereich zwischen 25 µl und 250 µl gewählt werden. In Abhängigkeit von der benutzten Enzymquelle werden Cofaktoren und Coenzyme zugefügt. Die Inkubation der Ansätze in dieser Mikrotiterplatte (Assay-MTP) gemäß Schritt (a) wird bei Raumtemperatur vorgenommen und beträgt je nach verwendeter Enzymaktivität (units) zwischen 5 und 60 Minuten. Zum Ende der Inkubation (Schritt (a)) wird die Platte in einen Zellharvester plaziert, der mit einer MTP bestückt ist, die eine Kationenaustauschermembran als Filterboden besitzt (Filter-MTP). Alle Ansätze der Assay-MTP werden in diese Filter-MTP überführt und über eine Kationenaustauscher-Filter-Platte, einen mit Phosphatgruppen beladenen Papierfilter, abgesaugt. Die Filter-MTP wird anschließend mit Puffer oder Wasser gewaschen. Mit Hilfe dieser Vorgehensweise wird das verbliebene Substrat Arginin auf dem Kationenaustauscher gebunden, während das enzymatisch gebildete radioaktive Citrullin quantitativ ausgewaschen wird. Nach Trocknen der Fitter-MTP und Zugabe von Szintillationsflüssigkeit kann das gebundene Arginin am Szintillationszähler ausgezählt werden. Eine nicht gehemmte NOS-Reaktion spiegelt sich in einer geringen Radioaktivität wieder. Eine gehemmte Enzymreaktion bedeutet, daß das radioaktive Arginin nicht umgesetzt worden ist. Das heißt, auf dem Filter befindet sich eine hohe Radioaktivität.

### Verwendete Materialien

- Arginin, L-[2, 3, 4-³H]-monohydrochlorid; Best.-Nr. NET-1123, Fa. NEN
- CaCl₂ wasserfrei; Best.- Nr. 2388.1000; Fa. Merck KGaA
- 1.4-Dithiothreitol (DTT), Best.-Nr. 708984; Fa. ROCHE
- Na₂EDTA-Dihydrat; Best.-Nr. 03680; Fa. FLUKA
- HEPES, Best:-Nr. H-3375; Fa. SIGMA
- NADPH, Tetranatriumsalz; Best.-Nr. 1585363; Fa. ROCHE
- TRIS; BEST.-Nr. 93349; Fa. FLUKA

- Enzym-Präparationspuffer:: 50 mM Tris-HCl mit 1 mM EDTA: Der pH-Wert des Puffers wurde bei 4 °C auf 7,4 eingestellt.
- Inkubationspuffer (-medium):: 50 mM HEPES mit 1 mM EDTA; 1,25 mM CaCl₂ und 1 mM Dithiothreitol. Der pH-Wert des Puffers wurde bei 25 °C auf 7,4 eingestellt.
- Waschmedium:: H₂O

### Enzympräparation

Als Ausgangsgewebe wurden Ratten-Cerebelli benutzt. Die Tiere wurden betäubt und getötet, das Gehirngewebe, das Cerebellum, wurde herauspräpariert, pro Rattenkleinhirn wurde 1 ml Enzympräparationspuffer (4 °C) hinzugegeben, und es wurde mit einem Polytron-Homogenisierer für 1 min bei 6000 U/min aufgeschlossen. Danach erfolgte Zentrifugation bei 4 °C für 15 min bei 20 000 g und anschließend Abdekantieren des Überstand und portioniertes Einfrieren bei - 80 °C (Verwerfen des Niederschlags).

### Inkubationsansatz:

Verwendet wurden 96-well MTP mit einer "Well"-Kapazität von ≤ 250 µl Pipettierreihenfolge: siehe Tabelle 1:

**Tabelle 1:**

| **Substanz** | **Molarität i.A.** | **µl** | *** Protein i.A:** |
|---|---|---|---|
| Inkubat.-Puffer | - | 100 | - |
| Testsubstanz | variabel; vorzugsweise 10⁻⁵M | variabel; vorzugsweise 20 µl | - |
| NADPH | 0,5 mM | 20 | - |
| Enzym (s. Beispiel 3) | - | variabel; maximales Volumen der Enzymlösung = 50 µl | variabel; maximale einsetzbare Proteinmenge = 100 µg |
| [³H]Substrat | variabel; vorzugsweise 50 nM | variabel; vorzugsweise 10 µl | |
| Endvolumen: | | max. 250 µl | |

| | | | |
|---|---|---|---|
| * Proteinbestimmung nach O.H. Lowry et al; J. Biol.Chem. **193,** 265 (1951) i.A. = im Ansatz | | | |

Nach beendetem Pipettiervorgang wurde ein Deckel auf diese MTP (Assay-MTP) gelegt. Inkubation bei 25 °C (Raumtemperatur (RT)) für 5-60 min, je nach Menge und Aktivität des eingesetzten Enzyms.

Anschließend wurde der Inhalt der Assay-MTP mit Hilfe eines 96-well Cell-Harvesters in eine 96-well Kationenaustauscher MTP (Filter-MTP) transferiert und abgesaugt. Es schloß sich eine einmalige Wäsche mit 200 ml H₂O (aus einer Wanne) an.

Dann wurde die Platte für 1 h bei 60 °C im Trockenschrank getrocknet. Dann wurde die Bodenseite der Filter-MTP von unten her exakt mit einem "back seal" versiegelt. Danach wurden pro well 35 µl Szintillator hinzupipettiert. Ferner wurde die Plattenoberseite mit einem "top seal" versiegelt. Nach 1 h Wartezeit wurde die Platte am β-Counter ausgemessen.

Im HTS-Betrieb wurden das Inkubationsmedium, NADPH- und Enzymlösung vor Beginn des Pipettierschrittes vereint, um nicht zeitaufwendig drei separate Pipettierungen vornehmen zu müssen.

Die Ergebnisse von Beispielverbindungen im NOS-Assay sind in Tabelle 3 wiedergegeben.

### Citrullin-Assay

Dieser Assay wurde wie von D. S. Bredt und S. H. Snyder (*Proc. Natl. Acad. Sci. USA* (1990), 87, 682-685) beschrieben durchgeführt. Die Ergebnisse von Beispielverbindungen im Citrullin-Assay sind in Tabelle 4 wiedergegeben.

Die folgenden Beispiele dienen der näheren Erläuterung der Erfindung, ohne sie darauf zu beschränken.

### Beispiele:

Die erfindungsgemnäßen Verbindungen wurden nach den folgenden allgemeinen Synthesevorschriften (AAV) hergestellt:

### Allgemeine Arbeitsvorschrift 1 (AAV 1)

Ein Rundbodenröhrchen aus Glas (Durchmesser 16 mm, Länge 125 mm) mit Gewinde wurde mit einem Rührer versehen und mit einem Schraubdeckel mit Septum verschlossen. Das Röhrchen wurde auf einen auf 15°C temperierten Reaktorblock gestellt. Es wurden nacheinander folgende Reagenzien hinzupipettiert:
1.) 1 ml einer 0,1 M Aminopyridin-II-Lösung, + 20 %ige HClO₄ in Dichlormethan
2.) 0,5 ml einer 0,3 M Lösung des Aldehyds III in Dichlormethan
3.) 0,575 ml einer 0,2 M *tert*.-Butyl-isonitril-Lösung in Dichlormethan

Das Reaktionsgemisch wurde bei 15°C 12 h lang gerührt. Danach wurde die Reaktionslösung abfiltriert. Das Röhrchen wurde dabei zweimal mit je 1 ml Dichlormethan und 200 µl Wasser gespült.

Das Reaktionsgemisch wurde mit 3 ml einer 10%igen NaCl-Lösung und 1,5 ml Dichlormethan versetzt und gründlich durchmischt. Die organische Phase wurde abgetrennt und die wäßrige Phase erneut mit 1,5 ml Dichlormethan extrahiert. Die vereinigten organischen Phasen wurden über 2,4 g MgSO₄ (granuliert) getrocknet. Das Lösungsmittel wurde in einer Vakuumzentrifuge entfernt.

Die eingesetzten Chemikalien und Lösungsmittel wurden kommerziell erworben. Jede Substanz wurde mit ESI-MS und/oder NMR analysiert.

Die gemäß AAV 1 hergestellten Beispiele 1-17 wurden im HTS-NOS-Assay automatisiert getestet. Die Ergebnisse sind in Tabelle 3 wiedergegeben.

**Tabelle 3**

| **Beispiel- Nr.** | **Verbindung** | **HTS-NOS- Assay: %- Hemmung (10 µM)** | **Masse berech- net** | **Masse gefun- den** |
|---|---|---|---|---|
| 1 | *tert*-Butyl-(7-methyl-2-pyridin-3-yl-imidazo[1,2-a]pyridin-3-yl)-amin | 63 | 280,37 | 281,3 |
| 2 | *tert*-Butyl-[2-(2,3-dimethoxy-phenyl)-5,7-dimethyl-imidazo[12-a]pyridin-3-yl]-amin | 61 | 353,46 | 354,2 |
| 3 | *tert*-Butyl-(2,5,7-trimethyl-imidazo[1,2-a]pyridin-3-yl)-amin | 67 | 231,34 | 232,2 |
| 4 | 3-(3-*tert*-Butylamino-57-dimethyl-imidazo[1,2-a]pyridin-2-yl)-phenol | 64 | 309,41 | 310,3 |
| 5 | *tert*-Butyl-(2-cyclohexyl-5,7-dimethyl-imidazo[1,2-a]pyridin-3-yl)-amin | 68 | 299,46 | 300,3 |
| 6 | 3-(3-*tert*-Butylamino-7-methyl-imidazo[1,2-a]pyridin-2-yl)-phenol | 59 | 295,38 | 296,3 |
| 7 | *tert*-Butyl-(2-furan-2-yl-57-dimethyl-imidazo[1,2-a]pyridin-3-yl)-amin | 60 | 283,37 | 284,2 |
| 8 | *tert*-Butyl-(7-methyl-2-naphthalen-1-yl-imidazo[1,2-a]pyridin-3-yl)-amin | 67 | 329,44 | 330,4 |
| 9 | *tert*-Butyl-[5,7-dimethyl-2-(5-nitro-furan-2-yl)-imidazo[1,2-a]pyridin-3-yl]-amin | 59 | 328,37 | 329,4 |
| 10 | [5-(3-*tert*-Butylamino-7-methyl-imidazo[1,2-a]pyridin-2-yl)-furan-2-yl]-methanol | 52 | 299,37 | 300,3 |
| 11 | *tert*-Butyl-[2-(5-[1,3]dioxolan-2-yl-furan-2-yl)-7-methyl-imidazo[1,2-a]pyridin-3-yl]-amin | 62 | 341,41 | 342,4 |
| 12 | 5-(3-tert-Butylamino-57-dimethyl-imidazo[1,2-a]pyridin-2-yl)-furan-2-carbonsäure | 56 | 327,38 | 328,3 |
| 13 | *tert*-Butyl-(2-furan-2-yl-7-methyl-imidazo[1,2-a]pyridin-3-yl)-amin | 57 | 269,34 | 270,4 |
| 14 | *tert*-Butyl-(7-methyl-2-pyridin-2-yl-imidazo[1,2-a]pyridin-3-yl)-amin | 67 | 280,37 | 281,3 |
| 15 | *tert*-Butyl-(2-cyclohexyl-7-methylimidazo[1,2-a]pyridin-3-yl)-amin | 52 | 285,43 | 286,4 |
| 16 | *tert*-Butyl-(2,7-dimethyl-imidazo[1,2-a]pyridin-3-yl)-amin | 65 | 217,31 | 218,2 |
| 17 | 5-(3*-tert*-Butylamino-7-methyl-imidazo[1,2-a]pyridin-2-yl)-thiophen-2-carbonsäure | 61 | 329,42 | 330,3 |

Als Vergleichsbeispiel wurde 7-Nitroindazol in dem NOS-Assay getestet mit einer Hemmung (10 µM) von 50%.

### Allgemeine Arbeitsvorschrift 2 (AAV 2)

### (Äquivalente bedeuten Stoffmengenäquivalente zum eingesetzten tert.-Butyl-isonitril IV):

In einem Reaktionsgefäß wurden zunächst 1,15 Äquivalente des Aminopyridins II in Dichlormethan (2 ml je mmol eingesetztem Isonitril IV) suspendiert bzw. gelöst. Hierzu wurden nacheinander 1,5 Äquivalente Aldehyd III, 1 Äquivalent *tert*.-Butyl-isonitril und schließlich wäßrige Perchlorsäurelösung (20 Massen-%: 0,098 ml je mmol eingesetztem Isonitril IV) zugegeben und der Ansatz für zwanzig Stunden bei Raumtemperatur gerührt.

Zur Aufarbeitung wurden gesättigte Natriumchloridlösung (ca. 5 ml je mmol eingesetztem Isonitril IV) und Dichlormethan (ca. 4 ml je mmol eingesetztem Isonitril IV) zugegeben, die Phasen getrennt und die organische Phase noch zweimal mit Dichlormethan (je ca. 2 ml je mmol eingesetztem Isonitril IV) extrahiert. Die vereinigten organischen Phasen wurden nacheinander mit Pufferlösung (pH 10; ca. 2 ml je mmol eingesetztem Isonitril IV) und ges. Natriumchloridlösung (ca. 2 ml je mmol eingesetztem Isonitril IV) gewaschen, über Natriumsulfat getrocknet, filtriert, am Rotationsverdampfer im Vakuum eingeengt und im Ölpumpenvakuum von Lösungsmittelresten befreit.

Das erhaltene Rohprodukt wurde entweder direkt einer Hydrochloridfällung zugeführt (Lösen der Rohbase in ca. 10 ml 2-Butanon je Gramm Base; Zugabe von 0,5 Mol-Äquivalenten Wasser, gefolgt von 1,1 Mol-Äquivalenten Chlortrimethylsilan (TMSCI) und Rühren über Nacht) oder mit Hexan (ca. 10 ml je mmol eingesetztem Isonitril) unter Rühren zum Rückfluß erhitzt. Löste sich das Rohprodukt nicht vollständig, wurde heiß abdekantiert. Nach Erkalten der Hexanlösung wurde ein gegebenenfalls erhaltener Feststoff abfiltriert und im Ölpumpenvakuum getrocknet. Das erhaltene Filtrat wurde am Rotationsverdampfer eingeengt und der Rückstand wiederum im Ölpumpenvakuum getrocknet. Auf diesem Wege wurden verschiedene Fraktionen erhalten:
0: Keine Behandlung mit Hexan
2: Aus Hexanlösung beim Erkalten ausgefallener Feststoff
4: Rückstand aus zur Trockne eingeengter Hexanlösung

Aus den jeweils erhaltenen Fraktionen wurden durch dünnschichtchromatographische und/oder NMR-spektroskopische Untersuchungen die Produktfraktion(en) identifiziert. Von einem Teil einer Produktfraktion wurde schließlich ein Hydrochlorid nach dem oben angegebenen Verfahren mit TMSCI gefällt.

Die erhaltenen Beispielsverbindungen 18-20 wurden im Citrullin-Assay auf NOS-Inhibition getestet. Die Ergebnisse sind in Tabelle 4 wiedergegeben.

**Tabelle 4**

| **Beispiel- Nr.** | **Verbindung** | **Ansatz** | **Ausbeute** | **Produkt- fraktion- (en)** | **Citrullin-Assay** |
|---|---|---|---|---|---|
| | | mmol Isonitril | g Produkt fraktion | | IC₅₀ (µM) |
| 18 | *tert*-Butyl-(2-furan-2-yl-5,7-imethyl-imidazo[1,2-]pyridin-3-yl)-amin; ydrochlorid | 18,8 | 4,64 | 0 | 2.8 |
| 19 | *tert*-Butyl-(7-methyl-2-phenyl-midazo[1,2-a]pyridin-3-yl)-min; Hydrochlorid | 54,1 | 9,06 | 2 | 2.4 |
| 20 | *tert*-Butyl-(2,5.7-trimethyl-midazo[1,2-a]pyridin-3-yl)-min; Hydrochlorid | 48,1 | 10,5 | 2 und 4 | 9,2 |

Als Vergleichsbeispiel wurden im Citrullin-Assay der aus dem Stand der Technik bekannte NOS-Inhibitor 7-Nitroindazol mit einem IC₅₀-Wert von 5,23 µM getestet.

### Pharmazeutische Formulierung eines erfindungsgemäßen Arzneimittels

1 g des Hydrochlorids von *tert*-Butyl-(2-furan-2-yl-57-dimethyl-imidazo[1,2-a]pyridin-3-yl)-amin wurde in 1 I Wasser für Injektionszwecke bei Raumtemperatur gelöst und anschließend durch Zugabe von Natriumchlorid auf isotone Bedingungen eingestellt.

## Patentansprüche

1. Verbindung der allgemeinen Struktur I 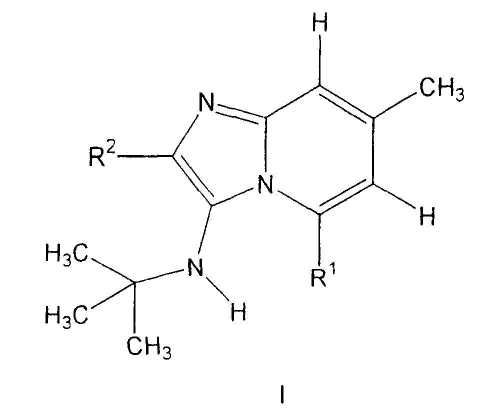 worin
R¹ H oder C₁₋₄-Alkanyl bedeutet, wobei Alkanyl geradkettig oder verzweigt ist und unsubstituiert oder ein- oder mehrfach substituiert ist, und
R² C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-C₃₋₈-Cycloalkyl, C₁₋₈-Alkyl-Heterocyclyl, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Cycloalkyl gesättigt oder ungesättigt ist und unsubstituiert oder ein- oder mehrfach substituiert, Heterocyclyl gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet,
in Form ihrer Base oder eines pharmazeutisch annehmbaren Salzes,
unter der Maßgabe, daß die Verbindung der allgemeinen Struktur I mit R¹ = Methyl und R² = Phenyl ausgenommen ist.

2. Verbindung nach Anspruch 1, wobei R¹ H oder Methyl bedeutet, in Form ihrer Base oder eines pharmazeutisch annehmbaren Salzes.

3. Verbindung nach einem der Ansprüche 1 oder 2, wobei R² Aryl oder Heteroaryl bedeutet, in Form ihrer Base oder eines pharmazeutisch annehmbaren Salzes.

4. Verbindung nach Anspruch 3, wobei R² 1-Naphthyl, Phenyl, Furyl, Thienyl oder Pyridinyl bedeutet und unsubstituiert oder ein- oder mehrfach substituiert ist, in Form ihrer Base oder eines pharmazeutisch annehmbaren Salzes.

5. Verbindung nach Anspruch 4, wobei 1-Naphthyl, Phenyl, Furyl, Thienyl oder Pyridinyl unsubstituiert oder mit -F, -CF₃, -OH, -OCH₃,-CH₂OH, -NO₂, -CO₂H und/oder -[1,3]-Dioxolan ein- oder zweifach substituiert ist, in Form ihrer Base oder eines pharmazeutisch annehmbaren Salzes.

6. Verbindung nach einem der Ansprüche 1 bis 5 in Form ihrer Base oder eines pharmazeutisch annehmbaren Salzes, insbesondere in Form ihres Hydrochlorids, die ausgewählt ist aus:
*tert*.-Butyl-(7-methyl-2-pyridin-3-yl-imidazo[1,2-a]pyridin-3-yl)-amin,
*tert*.-Butyl-[2-(23-dimethoxy-phenyl)-5,7-dimethyl-imidazo[1,2-a]pyridin-3-yl]-amin,
*tert*.-Butyl-(2,5,7-trimethyl-imidazo[1,2-a]pyridin-3-yl)-amin,
3-(3-*tert*.-Butylamino-5,7-dimethyl-imidazo[1,2-a]pyridin-2-yl)-phenol *tert*.-Butyl-(2-cyclohexyl-5,7-dimethyl-imidazo[1,2-a]pyridin-3-yl)-amin,
3-(3-*tert*.-Butylamino-7-methyl-imidazo[1,2-a]pyridin-2-yl)-phenol,
*tert*.-Butyl-(2-furan-2-yl-5,7-dimethyl-imidazo[1,2-a]pyridin-3-yl)-amin, *tert*.-Butyl-(7-methyl-2-naphthalen-1-yl-imidazo[1,2-a]pyridin-3-yl)-amin,
*tert.*-Butyl-[5,7-dimethyl-2-(5-nitro-furan-2-yl)-imidazo[1,2-a]pyridin-3-yl]-amin,
[5-(3-*tert*.-Butylamino-7-methyl-imidazo[1,2-a]pyridin-2-yl)-furan-2-yl]-methanol,
*tert.*-Butyl-[2-(5-[1,3]dioxolan-2-yl-furan-2-yl)-7-methyl-imidazo[1,2-a]pyridin-3-yl]-amin,
5-(3*-tert.*-Butylamino-5,7-dimethyl-imidazo[1,2-a]pyridin-2-yl)-furan-2-carbonsäure,
*tert.*-Butyl-(2-furan-2-yl-7-methyl-imidazo[1,2-a]pyridin-3-yl)-amin,
*tert.*-Butyl-(7-methyl-2-pyridin-2-yl-imidazo[1,2-a]pyridin-3-yl)-amin
*tert*.-Butyl-(2-cyclohexyl-7-methyl-imidazo[1,2-a]pyridin-3-yl)-amin,
*tert*.-Butyl-(27-dimethyl-imidazo[12-a]pyridin-3-yl)-amin,
5-(3-*tert*.-Butylamino-7-methyl-imidazo[1,2-a]pyridin-2-yl)-thiophen-2-carbonsäure,
*tert*.-Butyl-(7-methyl-2-phenyl-imidazo[1,2-a]pyridin-3-yl)-amin.

7. Verfahren zur Herstellung einer Verbindung der allgemeinen Struktur I nach einem der Ansprüche 1 bis 6 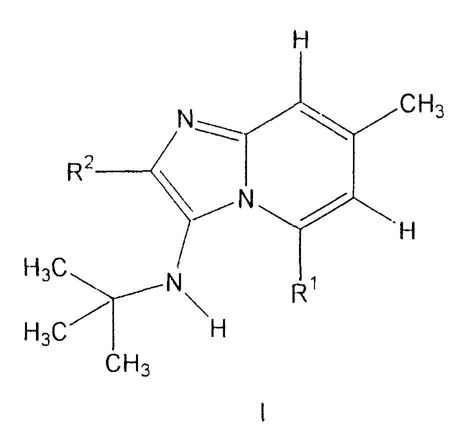 **dadurch gekennzeichnet, daß**
ein Aminopyridin der allgemeinen Struktur II 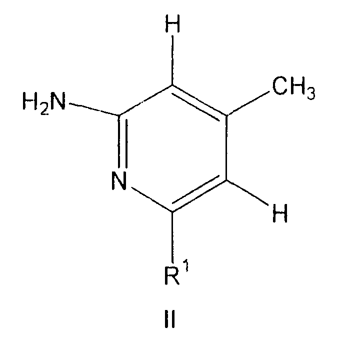 worin
R¹ H oder C₁₋₄-Alkanyl bedeutet, wobei Alkanyl geradkettig oder verzweigt ist und unsubstituiert oder ein- oder mehrfach substituiert ist,
mit einem Aldehyd der allgemeinen Struktur III 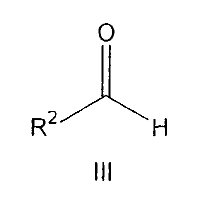 worin
R² C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-C₃₋₈-Cycloalkyl, C₁₋₈-Alkyl-Heterocyclyl, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Cycloalkyl gesättigt oder ungesättigt ist und unsubstituiert oder ein- oder mehrfach substituiert, Heterocyclyl gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet, unter der Maßgabe, daß R² nicht Phenyl bedeutet, wenn R¹ Methyl bedeutet,
und *tert*.-Butyl-isonitril der Struktur IV 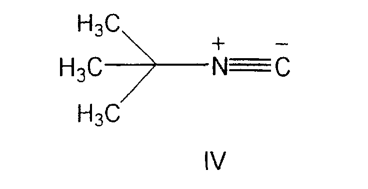 umgesetzt wird.

8. Verfahren nach Anspruch 7, **dadurch gekennzeichnet, daß** es in Gegenwart einer Säure, insbesondere Perchlorsäure, durchgeführt wird.

9. Verfahren nach einem der Ansprüche 7 oder 8, **dadurch gekennzeichnet, daß** die Umsetzung des Aminopyridins II mit dem Aldehyd III und dem *tert*.-Butyl-isonitril IV in einem Eintopfverfahren erfolgt.

10. Verfahren nach einem der Ansprüche 7 bis 9, **dadurch gekennzeichnet, daß** die gebildete Base der allgemeinen Struktur I durch Zugabe von Trimethylchlorsilan in ihr Hydrochlorid überführt wird.

11. Arzneimittel, enthaltend mindestens eine Verbindung der allgemeinen Struktur I 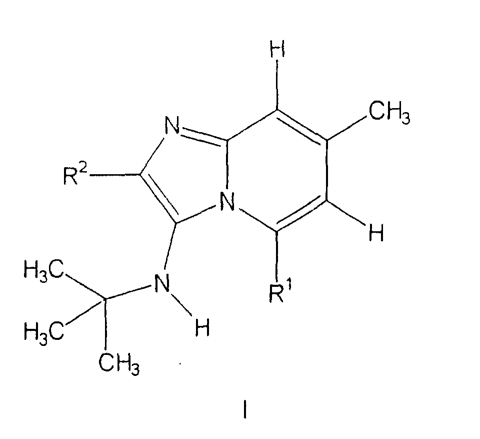 worin
R¹ H oder C₁₋₄-Alkanyl bedeutet, wobei Alkanyl geradkettig oder verzweigt ist und unsubstituiert oder ein- oder mehrfach substituiert ist, und
R² C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-C₃₋₈-Cycloalkyl, C₁₋₈-Alkyl-Heterocyclyl, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Cycloalkyl gesättigt oder ungesättigt ist und unsubstituiert oder ein- oder mehrfach substituiert, Heterocyclyl gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet,
in Form ihrer Base oder eines pharmazeutisch annehmbaren Salzes.

12. Verwendung einer Verbindung der allgemeinen Struktur I 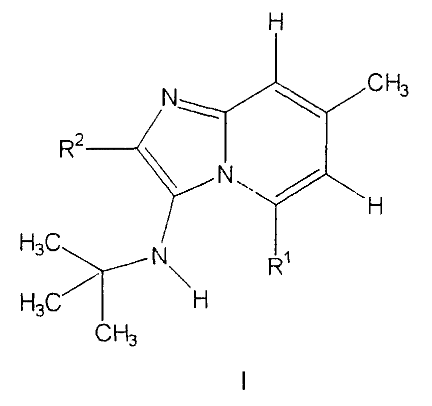 worin
R¹ H oder C₁₋₄-Alkanyl bedeutet, wobei Alkanyl geradkettig oder verzweigt ist und unsubstituiert oder ein- oder mehrfach substituiert ist, und
R² C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-C₃₋₈-Cycloalkyl, C₁₋₈-Alkyl-Heterocyclyl, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Cycloalkyl gesättigt oder ungesättigt ist und unsubstituiert oder ein- oder mehrfach substituiert, Heterocyclyl gesättigt oder ungesättigt ist und unsubstituiert oder ein- oder mehrfach substituiert ist. Aryl unsubstituiert oder ein- oder mehrfach substituiert ist und Heteroaryl unsubstituiert oder ein- oder mehrfach substituiert ist, bedeutet,
in Form ihrer Base oder eines pharmazeutisch annehmbaren Salzes, zur Herstellung eines Medikaments zur NO-Synthase-Inhibierung.

13. Verwendung nach Anspruch 12, **dadurch gekennzeichnet, daß** die Verbindung der allgemeinen Struktur I ausgewählt ist aus:
*tert*.-Butyl-(7-methyl-2-pyridin-3-yl-imidazo[1,2-a]pyridin-3-yl)-amin,
*tert*.-Butyl-[2-(2,3-dimethoxy-phenyl)-5,7-dimethyl-imidazo[1,2-a]pyridin-3-yl]-amin,
*tert*.-Butyl-(2,5,7-trimethyl-imidazo[1,2-a]pyridin-3-yl)-amin,
3-(3-*tert*.-Butylamino-5,7-dimethyl-imidazo[1,2-a]pyridin-2-yl)-phenol *tert*.-Butyl-(2-cyclohexyl-5,7-dimethyl-imidazo[1,2-a]pyridin-3-yl)-amin,
3-(3-*tert*.-Butylamino-7-methyl-imidazo[1,2-a]pyridin-2-yl)-phenol,
*tert*.-Butyl-(2-furan-2-yl-5,7-dimethyl-imidazo[1,2-a]pyridin-3-yl)-amin,
*tert*.-Butyl-(7-methyl-2-naphthalen-1-yl-imidazo[1,2-a]pyridin-3-yl)-amin,
*tert*.-Butyl-[5,7-dimethyl-2-(5-nitro-furan-2-yl)-imidazo[1,2-a]pyridin-3-yl]-amin,
[5-(3*-tert.*-Butylamino-7-methyl-imidazo[1,2-a]pyridin-2-yl)-furan-2-yl]-methanol,
*tert*.-Butyl-[2-(5-[1,3]dioxolan-2-yl-furan-2-yl)-7-methyl-imidazo[12-a]pyridin-3-yl]-amin,
5-(3-*tert*.-Butylamino-5,7-dimethyl-imidazo[1,2-a]pyridin-2-yl)-furan-2-carbonsäure,
*tert*.-Butyl-(2-furan-2-yl-7-methyl-imidazo[1,2-a]pyridin-3-yl)-amin,
*tert*.-Butyl-(7-methyl-2-pyridin-2-yl-imidazo[1,2-a]pyridin-3-yl)-amin,
*tert*.-Butyl-(2-cyclohexyl-7-methyl-imidazo[1,2-a]pyridin-3-yl)-amin,
*tert*.-Butyl-(2,7-dimethyl-imidazo[1,2-a]pyridin-3-yl)-amin,
5-(3-*tert*.-Butylamino-7-methyl-imidazo[1,2-a]pyridin-2-yl)-thiophen-2-carbonsäure,
*tert*.-Butyl-(7-methyl-2-phenyl-imidazo[1,2-a]pyridin-3-yl)-amin.

14. Verwendung einer Verbindung der allgemeinen Struktur I 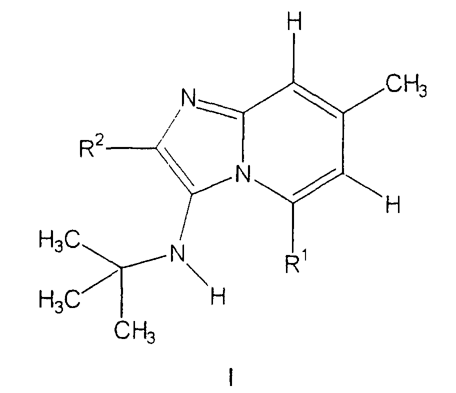 worin
R¹ H oder C₁₋₄-Alkanyl bedeutet, wobei Alkanyl geradkettig oder verzweigt ist und unsubstituiert oder ein- oder mehrfach substituiert ist, und
R² C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-C₃₋₈-Cycloalkyl, C₁₋₈-Alkyl-Heterocyclyl, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Cycloalkyl gesättigt oder ungesättigt ist und unsubstituiert oder ein- oder mehrfach substituiert, Heterocyclyl gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet,
in Form ihrer Base oder eines pharmazeutisch annehmbaren Salzes, zur Herstellung eines Medikaments zur Behandlung von Migräne.

15. Verwendung nach Anspruch 14, **dadurch gekennzeichnet, daß** die Verbindung der allgemeinen Struktur I ausgewählt ist aus
*tert*.-Butyl-(7-methyl-2-pyridin-3-yl-imidazo[1,2-a]pyridin-3-yl)-amin
*tert*.-Butyl-[2-(2,3-dimethoxy-phenyl)-5,7-dimethyl-imidazo[1,2-a]pyridin-3-yl]-amin,
*tert*.-Butyl-(2,5,7-trimethyl-imidazo[1,2-a]pyridin-3-yl)-amin,
3-(3-*tert*.-Butylamino-5,7-dimethyl-imidazo[1,2-a]pyridin-2-yl)-phenol *tert*.-Butyl-(2-cyclohexyl-5,7-dimethyl-imidazo[1,2-a]pyridin-3-yl)-amin.
3-(3-*tert*.-Butylamino-7-methyl-imidazo[1,2-a]pyridin-2-yl)-phenol,
*tert*.-Butyl-(2-furan-2-yl-5,7-dimethyl-imidazo[1,2-a]pyridin-3-yl)-amin,
*tert*.-Butyl-(7-methyl-2-naphthalen-1-yl-imidazo[1,2-a]pyridin-3-yl)-amin,
*tert*.-Butyl-[5,7-dimethyl-2-(5-nitro-furan-2-yl)-imidazo[1,2-a]pyridin-3-yl]-amin,
[5-(3-*tert*.-Butylamino-7-methyl-imidazo[1,2-a]pyridin-2-yl)-furan-2-yl]-methanol,
*tert*.-Butyl-[2-(5-[1,3]dioxolan-2-yl-furan-2-yl)-7-methyl-imidazo[1,2-a]pyridin-3-yl]-amin,
5-(3*-tert*.-Butylamino-5,7-dimethyl-imidazo[1,2-a]pyridin-2-yl)-furan-2-carbonsäure,
*tert.*-Butyl-(2-furan-2-yl-7-methyl-imidazo[1,2-a]pyridin-3-yl)-amin,
*tert*.-Butyl-(7-methyl-2-pyridin-2-yl-imidazo[1,2-a]pyridin-3-yl)-amin,
*tert*.-Butyl-(2-cyclohexyl-7-methyl-imidazo[1,2-a]pyridin-3-yl)-amin.
*tert*.-Butyl-(2,7-dimethyl-imidazo[1,2-a]pyridin-3-yl)-amin,
5-(3-*tert*.-Butylamino-7-methyl-imidazo[1,2-a]pyridin-2-yl)-thiophen-2-carbonsäure,
*tert*.-Butyl-(7-methyl-2-phenyl-imidazo[1,2-a]pyridin-3-yl)-amin.

16. Verwendung einer Verbindung der allgemeinen Struktur I 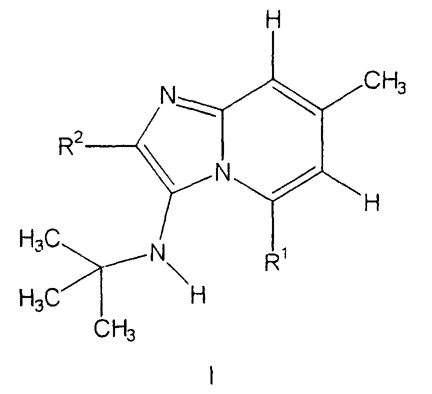 worin
R¹ H oder C₁₋₄-Alkanyl bedeutet, wobei Alkanyl geradkettig oder verzweigt ist und unsubstituiert oder ein- oder mehrfach substituiert ist, und
R² C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-C₃₋₈-Cycloalkyl, C₁₋₈-Alkyl-Heterocyclyl, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Cycloalkyl gesättigt oder ungesättigt ist und unsubstituiert oder ein- oder mehrfach substituiert, Heterocyclyl gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet,
in Form ihrer Base oder eines pharmazeutisch annehmbaren Salzes, zur Herstellung eines Medikaments zur Behandlung von septischem Schock, Multipler Sklerose, Morbus Parkinson, Morbus Alzheimer, Morbus Huntington, Entzündungen, Entzündungsschmerz, cerebraler Ischämie, Diabetes, Meningitis, Arteriosklerose und/oder für die Wundheilung.

17. Pharmazeutische Zusammensetzung, die mindestens eine Verbindung der allgemeinen Struktur I 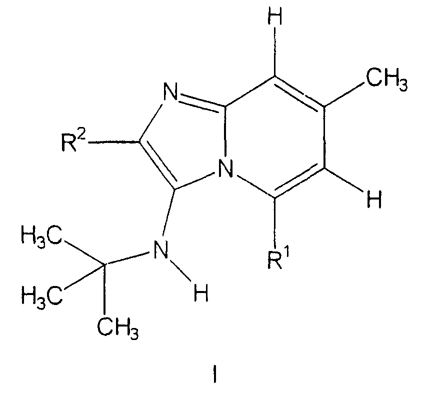 worin
R¹ H oder C₁₋₄-Alkanyl bedeutet, wobei Alkanyl geradkettig oder verzweigt ist und unsubstituiert oder ein- oder mehrfach substituiert ist, und
R² C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-C₃₋₈-Cycloalkyl, C₁₋₈-Alkyl-Heterocyclyl. C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Cycloalkyl gesättigt oder ungesättigt ist und unsubstituiert oder ein- oder mehrfach substituiert, Heterocyclyl gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet,
in Form ihrer Base oder eines pharmazeutisch annehmbaren Salzes, sowie mindestens einen pharmazeutischen Hilfsstoff enthält.

18. Verwendung eines Erzeugnisses zur Herstellung eines Mittels zur NO-Synthase-Inhibierung und/oder zur Behandlung von Migräne und/oder zur Behandlung von septischem Schock, Multipler Sklerose, Morbus Parkinson, Morbus Alzheimer, Morbus Huntington, Entzündungen, Entzündungsschmerz, cerebraler Ischämie, Diabetes, Meningitis, Arteriosklerose und/oder für die Wundheilung mit einer Verbindung der allgemeinen Struktur I oder eines pharmazeutisch annehmbaren Salzes davon 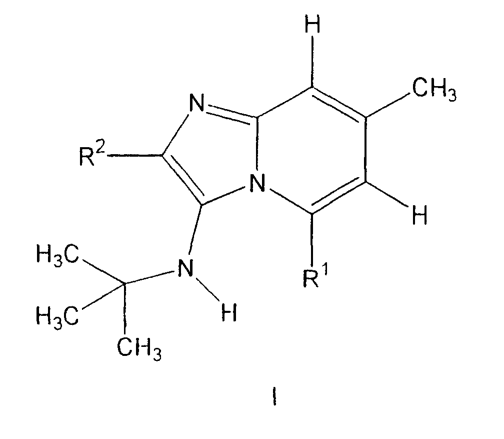 worin
R¹ H oder C₁₋₄-Alkanyl bedeutet, wobei Alkanyl geradkettig oder verzweigt ist und unsubstituiert oder ein- oder mehrfach substituiert ist, und
R² C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-C₃₋₈-Cycloalkyl, C₁₋₈-Alkyl-Heterocyclyl, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Cycloalkyl gesättigt oder ungesättigt ist und unsubstituiert oder ein- oder mehrfach substituiert, Heterocyclyl gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet.

19. Verwendung eines Erzeugnisses zur Herstellung eines Mittels zur NO-Synthase-Inhibierung und/oder zur Behandlung von Migräne und/oder zur Behandlung von septischem Schock, Multipler Sklerose, Morbus Parkinson, Morbus Alzheimer, Morbus Huntington, Entzündungen, Entzündungsschmerz, cerebraler Ischämie, Diabetes, Meningitis, Arteriosklerose und/oder für die Wundheilung in/an einem Säugetier, insbesondere einem Menschen, **dadurch gekennzeichnet, daß** eine wirksame Menge einer Verbindung der allgemeinen Struktur I oder eines pharmazeutisch annehmbaren Salzes davon 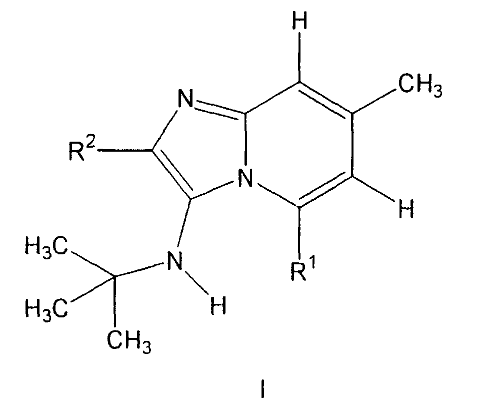 worin
R¹ H oder C₁₋₄-Alkanyl bedeutet, wobei Alkanyl geradkettig oder verzweigt ist und unsubstituiert oder ein- oder mehrfach substituiert ist, und
R² C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist. Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-C₃₋₈-Cycloalkyl, C₁₋₈-Alkyl-Heterocyclyl, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Cycloalkyl gesättigt oder ungesättigt ist und unsubstituiert oder ein- oder mehrfach substituiert. Heterocyclyl gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet, verwendet wird.

## Claims

1. A compound of general structure I 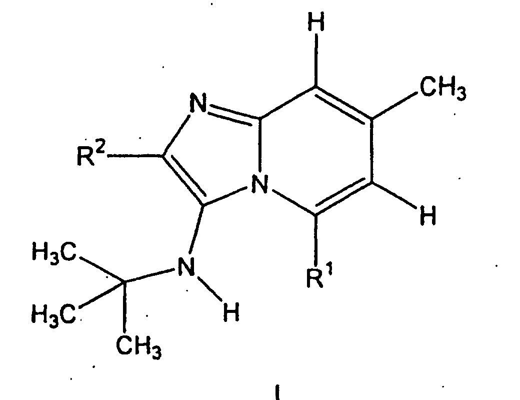 wherein
R¹ denotes H or a C₁₋₄ alkanyl, wherein the alkanyl is straight-chain or branched and is unsubstituted or singly or multiply substituted, and
R² denotes a C₁₋₈ alkyl, wherein the alkyl is straight-chain or branched, is saturated or unsaturated and is unsubstituted or singly or multiply substituted, a C₃₋₈ cycloalkyl, wherein the cycloalkyl is saturated or unsaturated and is unsubstituted or singly or multiply substituted, a heterocyclyl, wherein the heterocyclyl is saturated or unsaturated and is unsubstituted or singly or multiply substituted, an aryl, wherein the aryl is unsubstituted or singly or multiply substituted, a heteroaryl, wherein the heteroaryl is unsubstituted or singly or multiply substituted, a C₁₋₈ alkyl-C₃₋₈ cycloalkyl, a C₁₋₈ alkyl-heterocyclyl, a C₁₋₈ alkyl-aryl or a C₁₋₈ alkyl-heteroaryl, wherein the alkyl is straight-chain or branched, is saturated or unsaturated and is unsubstituted or singly or multiply substituted, the cycloalkyl is saturated or unsaturated and is unsubstituted or singly or multiply substituted, the heterocyclyl is saturated or unsaturated and is unsubstituted or singly or multiply substituted, the aryl is unsubstituted or singly or multiply substituted, and the heteroaryl is unsubstituted or singly or multiply substituted,
in the form of its base or of a pharmaceutically acceptable salt,
with the proviso that the compound of general structure I where R¹ = methyl and R² = phenyl is excluded.

2. A compound according to claim 1, wherein R¹ denotes H or methyl, in the form of its base or of a pharmaceutically acceptable salt.

3. A compound according to either one of claims 1 or 2, wherein R² denotes aryl or heteroaryl, in the form of its base or of a pharmaceutically acceptable salt.

4. A compound according to claim 3, wherein R² denotes 1-naphthyl, phenyl, furyl, thienyl or pyridinyl and is unsubstituted or singly or multiply substituted, in the form of its base or of a pharmaceutically acceptable salt.

5. A compound according to claim 4, wherein 1-naphthyl, phenyl, furyl, thienyl or pyridinyl is unsubstituted or is singly or doubly substituted with -F, -CF₃, -OH, -OCH₃, -CH₂OH, -NO₂, -CO₂ and/or -[1,3]-dioxolane, in the form of its base or of a pharmaceutically acceptable salt.

6. A compound according to any one of claims 1 to 5 in the form of its base or of a pharmaceutically acceptable salt, particularly in the form of its hydrochloride, which is selected from
*tert.*-butyl-(7-methyl-2-pyridin-3-yl-imidazo[1,2-a]pyridin-3-yl)-amine,
*tert*.-butyl-[2-(2,3-dimethoxy-phenyl)-5,7-dimethyl-imidazo[1,2-a]pyridin-3-yl]-amine,
*tert*-butyl-(2,5,7-tri-methyl-imidazo[1,2-a]pyridin-3-yl)-amine,
3-(3-tert.-butylamino-5,7-dimethyl-imidazo[1,2-a]pyridin-2-yl)-phenol
*tert*.-butyl-(2-cyclohexyl-5,7-dimethyl-imidazo[1,2-a]pyridin-3-yl)-amine,
3-(3-*tert*.-butylamino-7-methyl-imidazo[1,2-a]pyridin-2-yl)-phenol,
*tert*.-butyl-(2-furan-2-yl-5,7-dimethyl-imidazo[1,2-a]pyridin-3-yl)-amine,
*tert.*-butyl-(7-methyl-2-naphthalen-1-yl-imidazo[1,2-a]pyridin-3-yl) amine,
tert.-butyl-(5,7-dimethyl-2-(5-nitro-furan-2-yl)-imidazo[1,2-a]pyridin-3-yl]-amine,
[5-(3-tert.-butylamino-7-methyl-imidazo[1,2-a]pyridin-2-yl)-furan-2-yl]-methanol,
tert.-butyl-[2-(5-[1,3]dioxolan-2-yl-furan-2-yl)-7-methyl-imidazo[1,2-a]-pyridin-3-yl]-amine,
5-(3-*tert*.-butylamino-5,7-dimethyl-imidazo[1,2-a]pyridin-2-yl)-furan-2-carboxylic acid,
*tert*.-butyl-(2-furan-2-yl-7-methyl-imidazo[1,2-a]pyridin-3-yl)-amine,
tert.-butyl- (7-methyl-2-pyridin-2-yl-imidazo[1,2-a]pyridin-3-yl)-amine,
*tert*.-butyl-(2-cyclohexyl-7-methyl-imidazo[1,2-a]pyridin-3-yl)-amine,
tert.-butyl-(2,7-dimethyl-imidazo[1,2-a]pyridin-3-yl)-amine,
5-(3-tert.-butylamino-7-methyl-imidazo[1,2-a]pyridin-2-yl)-thiophene-2-carboxylic acid,
*tert.*-butyl-(7-methyl-2-phenyl-imidazo[1,2-a]pyridin-3-yl)-amine.

7. A method of producing a compound of general structure I according to any one of claims 1 to 6 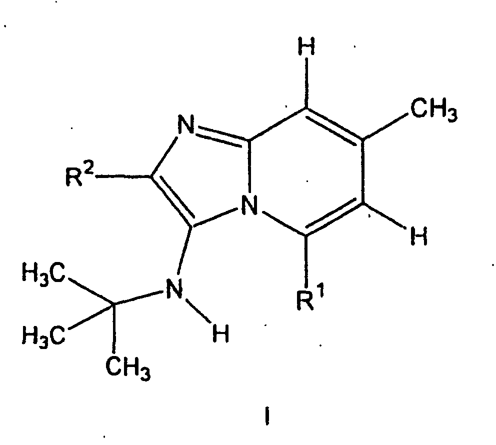 **characterised in that**
an aminopyridine of general structure II 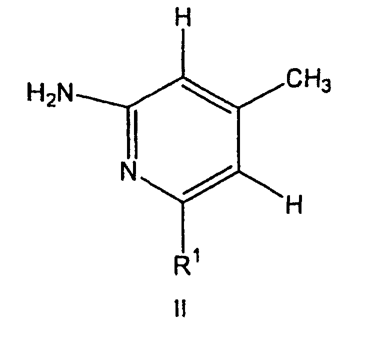 wherein
R¹ denotes H or a C₁₋₄ alkanyl, wherein the alkanyl is straight-chain or branched and is unsubstituted or singly or multiply substituted,
is reacted with an aldehyde of general structure III 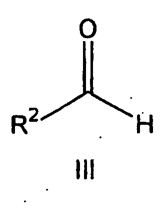 wherein
R² denotes a C₁₋₈ alkyl, wherein the alkyl is straight-chain or branched, is saturated or unsaturated and is unsubstituted or singly or multiply substituted, a C₃₋₈ cycloalkyl, wherein the cycloalkyl is saturated or unsaturated and is unsubstituted or singly or multiply substituted, a heterocyclyl, wherein the heterocyclyl is saturated or unsaturated and is unsubstituted or singly or multiply substituted, an aryl, wherein the aryl is unsubstituted or singly or multiply substituted, a heteroaryl, wherein the heteroaryl is unsubstituted or singly or multiply substituted, a C₁₋₈ alkyl-C₃₋₈ cycloalkyl, a C₁₋₈ alkyl-heterocyclyl, a C₁₋₈ alkyl-aryl or a C₁₋₈ alkyl-heteroaryl, wherein the alkyl is straight-chain or branched, is saturated or unsaturated and is unsubstituted or singly or multiply substituted, the cycloalkyl is saturated or unsaturated and is unsubstituted or singly or multiply substituted, the heterocyclyl is saturated or unsaturated and is unsubstituted or singly or multiply substituted, the aryl is unsubstituted or singly or multiply substituted, and the heteroaryl is unsubstituted or singly or multiply substituted, with the proviso that R² does not denote phenyl if R¹ denotes methyl,
and with *tert*.-butyl isonitrile of structure IV

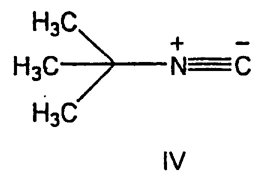

8. A method according to claim 7, **characterised in that** it is carried out in the presence of an acid, particularly perchloric acid.

9. A method according to either one of claims 7 or 8, **characterised in that** the reaction of aminopyridine II with aldehyde III and with *tert.*-butyl isonitrile IV is conducted as a one-pot process.

10. A method according to any one of claims 7 to 9, **characterised in that** the base of general structure I which is formed is converted into its hydrochloride by the addition of trimethylchlorosilane.

11. A medicament containing at least one compound of general structure I 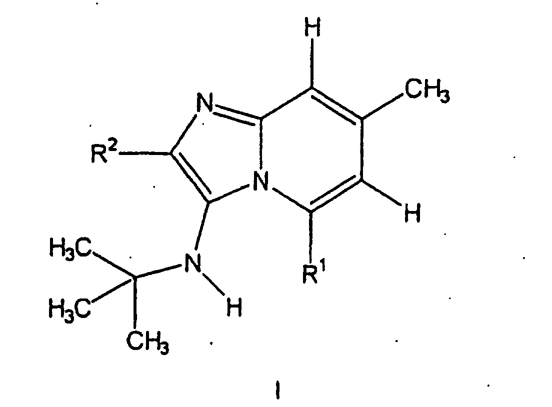 wherein
R¹ denotes H or a C₁₋₄ alkanyl, wherein the alkanyl is straight-chain or branched and is unsubstituted or singly or multiply substituted, and
R² denotes a C₁₋₈ alkyl, wherein the alkyl is straight-chain or branched, is saturated or unsaturated and is unsubstituted or singly or multiply substituted, a C₃₋₈ cycloalkyl, wherein the cycloalkyl is saturated or unsaturated and is unsubstituted or singly or multiply substituted, a heterocyclyl, wherein the heterocyclyl is saturated or unsaturated and is unsubstituted or singly or multiply substituted, an aryl, wherein the aryl is unsubstituted or singly or multiply substituted, a heteroaryl, wherein the heteroaryl is unsubstituted or singly or multiply substituted, a C₁₋₈ alkyl-C₃₋₈ cycloalkyl, a C₁₋₈ alkyl-heterocyclyl, a C₁₋₈ alkyl-aryl or a C₁₋₈ alkyl-heteroaryl, wherein the alkyl is straight-chain or branched, is saturated or unsaturated and is unsubstituted or singly or multiply substituted, the cycloalkyl is saturated or unsaturated and is unsubstituted or singly or multiply substituted, the heterocyclyl is saturated or unsaturated and is unsubstituted or singly or multiply substituted, the aryl is unsubstituted or singly or multiply substituted, and the heteroaryl is unsubstituted or singly or multiply substituted,
in the form of its base or of a pharmaceutically acceptable salt.

12. Use of a compound of general structure I 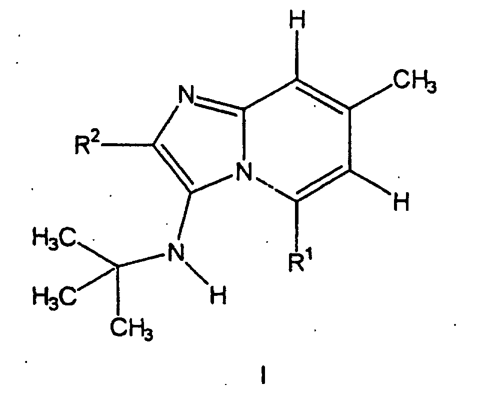 wherein
R¹ denotes H or a C₁₋₄ alkanyl, wherein the alkanyl is straight-chain or branched and is unsubstituted or singly or multiply substituted, and
R² denotes a C₁₋₈ alkyl, wherein the alkyl is straight-chain or branched, is saturated or unsaturated and is unsubstituted or singly or multiply substituted, a C₃₋₈ cycloalkyl, wherein the cycloalkyl is saturated or unsaturated and is unsubstituted or singly or multiply substituted, a heterocyclyl, wherein the heterocyclyl is saturated or unsaturated and is unsubstituted or singly or multiply substituted, an aryl, wherein the aryl is unsubstituted or singly or multiply substituted, a heteroaryl, wherein the heteroaryl is unsubstituted or singly or multiply substituted, a C₁₋₈ alkyl-C₃₋₈ cycloalkyl, a C₁₋₈ alkyl-heterocyclyl, a C₁₋₈ alkyl-aryl or a C₁₋₈ alkyl-heteroaryl, wherein the alkyl is straight-chain or branched, is saturated or unsaturated and is unsubstituted or singly or multiply substituted, the cycloalkyl is saturated or unsaturated and is unsubstituted or singly or multiply substituted, the heterocyclyl is saturated or unsaturated and is unsubstituted or singly or multiply substituted, the aryl is unsubstituted or singly or multiply substituted, and the heteroaryl is unsubstituted or singly or multiply substituted,
in the form of its base or of a pharmaceutically acceptable salt,
for producing a medicament for inhibiting NO synthase.

13. A use according to claim 12, **characterised in that** the compound of general structure I is selected from
*tert.*-butyl-(7-methyl-2-pyridin-3-yl-imidazo[1,2-a]pyridin-3-yl)-amine,
*tert.*-butyl-[2-(2,3-dimethoxy-phenyl)-5,7-dimethyl-imidazo[1,2-a]pyridin-3-yl]-amine,
tert-butyl-(2,5,7-tri-methyl-imidazo[1,2-a]pyridin-3-yl)-amine,
3-(3*-tert.*-butylamino-5,7-dimethyl-imidazo[1,2-a]pyridin-2-yl)-phenol
*tert*.-butyl-(2-cyclohexyl-5,7-dimethyl-imidazo[1,2-a]pyridin-3-yl)-amine,
3-(3-*tert*.-butylamino-7-methyl-imidazo[1,2-a]pyridin-2-yl)-phenol,
*tert*.-butyl-(2-furan-2-yl-5,7-dimethyl-imidazo[1,2-a]pyridin-3-yl)-amine,
*tert*.-butyl-(7-methyl-2-naphthalen-1-yl-imidazo[1,2-a]pyridin-3-yl) amine,
*tert*.-butyl-(5,7-dimethyl-2-(5-nitro-furan-2-yl)-imidazo[1,2-a]pyridin-3-yl]-amine,
[5-(3-*tert*.-butylamino-7-methyl-imidazo[1,2-a]pyridin-2-yl)-furan-2-yl]-methanol,
*tert*.-butyl-[2-(5-[1,3]dioxolan-2-yl-furan-2-yl)-7-methyl-imidazo[1,2-a]-pyridin-3-yl]-amine,
5-(3-*tert*.-butylamino-5,7-dimethyl-imidazo[1,2-a]pyridin-2-yl)-furan-2-carboxylic acid,
*tert*.-butyl-(2-furan-2-yl-7-methyl-imidazo[1,2-a]pyridin-3-yl)-amine,
tert.-butyl-(7-methyl-2-pyridin-2-yl-imidazo[1,2-a]pyridin-3-yl)-amine,
tert.-butyl-(2-cyclohexyl-7-methyl-imidazo[1,2-a]pyridin-3-yl)-amine,
*tert*.-butyl-(2,7-dimethyl-imidazo[1,2-a]pyridin-3-yl)-amine,
5-(3-*tert*.-butylamino-7-methyl-imidazo[1,2-a]pyridin-2-yl)-thiophene-2-carboxylic acid,
tert.-butyl- (7-methyl-2-phenyl-imidazo[1,2-a]pyridin-3-yl)-amine.

14. Use of a compound of general structure I 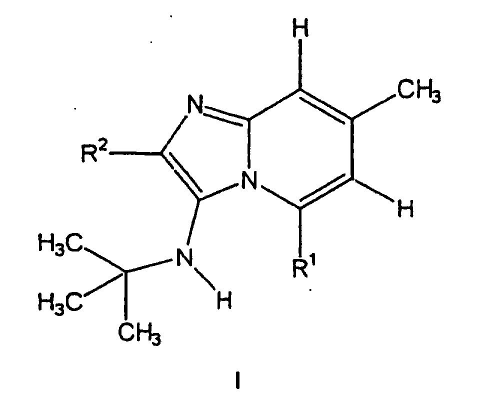 wherein
R¹ denotes H or a C₁₋₄ alkanyl, wherein the alkanyl is straight-chain or branched and is unsubstituted or singly or multiply substituted, and
R² denotes a C₁₋₈ alkyl, wherein the alkyl is straight-chain or branched, is saturated or unsaturated and is unsubstituted or singly or multiply substituted, a C₃₋₈ cycloalkyl, wherein the cycloalkyl is saturated or unsaturated and is unsubstituted or singly or multiply substituted, a heterocyclyl, wherein the heterocyclyl is saturated or unsaturated and is unsubstituted or singly or multiply substituted, an aryl, wherein the aryl is unsubstituted or singly or multiply substituted, a heteroaryl, wherein the heteroaryl is unsubstituted or singly or multiply substituted, a C₁₋₈ alkyl-C₃₋₈ cycloalkyl, a C₁₋₈ alkyl-heterocyclyl, a C₁₋₈ alkyl-aryl or a C₁₋₈ alkyl-heteroaryl, wherein the alkyl is straight-chain or branched, is saturated or unsaturated and is unsubstituted or singly or multiply substituted, the cycloalkyl is saturated or unsaturated and is unsubstituted or singly or multiply substituted, the heterocyclyl is saturated or unsaturated and is unsubstituted or singly or multiply substituted, the aryl is unsubstituted or singly or multiply substituted, and the heteroaryl is unsubstituted or singly or multiply substituted,
in the form of its base or of a pharmaceutically acceptable salt,
for producing a medicament for the treatment of migraine.

15. A use according to claim 14, **characterised in that** the compound of general structure I is selected from
*tert*.-butyl-(7-methyl-2-pyridin-3-yl-imidazo[1,2-a]pyridin-3-yl)-amine,
*tert*.-butyl- [2-(2,3-dimethoxy-phenyl)-5,7-dimethylimidazo[1,2-a]pyridin-3-yl]-amine,
*tert*-butyl-(2,5,7-tri-methyl-imidazo[1,2-a]pyridin-3-yl)-amine,
3-(3-tert.-butylamino-5,7-dimethyl-imidazo[1,2-a]pyridin-2-yl)-phenol
*tert*.-butyl-(2-cyclohexyl-5,7-dimethyl-imidazo[1,2-a]pyridin-3-yl)-amine,
3-(3*-tert.*-butylamino-7-methyl-imidazo[1,2-a]pyridin-2-yl)-phenol,
*tert*.-butyl-(2-furan-2-yl-5,7-dimethyl-imidazo[1,2-a]pyridin-3-yl)-amine,
*tert.*-butyl-(7-methyl-2-naphthalen-1-yl-imidazo[1,2-a]pyridin-3-yl) amine,
*tert*.-butyl-(5,7-dimethyl-2-(5-nitro-furan-2-yl)-imidazo[1,2-a]pyridin-3-yl]-amine,
[5-(3-tert.-butylamino-7-methyl-imidazo[1,2-a]pyridin-2-yl)-furan-2-yl]-methanol,
*tert*.-butyl-[2-(5-[1,3] dioxolan-2-yl-furan-2-yl)-7-methyl-imidazo[1,2-a]-pyridin-3-yl]-amine,
5-(3-*tert*.-butylamino-5,7-dimethyl-imidazo[1,2-a]pyridin-2-yl)-furan-2-carboxylic acid,
*tert*.-butyl-(2-furan-2-yl-7-methyl-imidazo[1,2-a]pyridin-3-yl)-amine,
*tert*.-butyl-(7-methyl-2-pyridin-2-yl-imidazo[1,2-a]pyridin-3-yl)-amine,
*tert*.-butyl-(2-cyclohexyl-7-methyl-imidazo[1,2-a]pyridin-3-yl)-amine,
tert.-butyl-(2,7-dimethyl-imidazo[1,2-a]pyridin-3-yl)-amine,
5-(3-*tert*.-butylamino-7-methyl-imidazo[1,2-a]pyridin-2-yl)-thiophene-2-carboxylic acid,
tert.-butyl-(7-methyl-2-phenyl-imidazo[1,2-a]pyridin-3-yl)-amine.

16. Use of a compound of general structure I 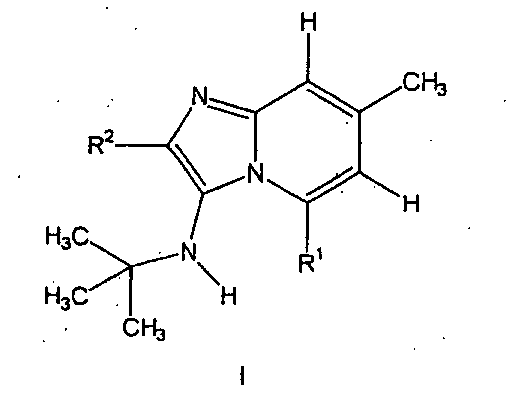 wherein
R¹ denotes H or a C₁₋₄ alkanyl, wherein the alkanyl is straight-chain or branched and is unsubstituted or singly or multiply substituted, and
R² denotes a C₁₋₈ alkyl, wherein the alkyl is straight-chain or branched, is saturated or unsaturated and is unsubstituted or singly or multiply substituted, a C₃₋₈ cycloalkyl, wherein the cycloalkyl is saturated or unsaturated and is unsubstituted or singly or multiply substituted, a heterocyclyl, wherein the heterocyclyl is saturated or unsaturated and is unsubstituted or singly or multiply substituted, an aryl, wherein the aryl is unsubstituted or singly or multiply substituted, a heteroaryl, wherein the heteroaryl is unsubstituted or singly or multiply substituted, a C₁₋₈ alkyl-C₃₋₈ cycloalkyl, a C₁₋₈ alkyl-heterocyclyl, a C₁₋₈ alkyl-aryl or a C₁₋₈ alkyl-heteroaryl, wherein the alkyl is straight-chain or branched, is saturated or unsaturated and is unsubstituted or singly or multiply substituted, the cycloalkyl is saturated or unsaturated and is unsubstituted or singly or multiply substituted, the heterocyclyl is saturated or unsaturated and is unsubstituted or singly or multiply substituted, the aryl is unsubstituted or singly or multiply substituted, and the heteroaryl is unsubstituted or singly or multiply substituted,
in the form of its base or of a pharmaceutically acceptable salt,
for producing a medicament for the treatment of septic shock, multiple sclerosis, Parkinson's medical condition, Alzheimer's medical condition,
Huntington's medical condition, inflammation, pain due to inflammation, cerebral ischaemia, diabetes, meningitis, arteriosclerosis and/or for the healing of wounds.

17. A pharmaceutical composition which contains at least one compound of general structure I 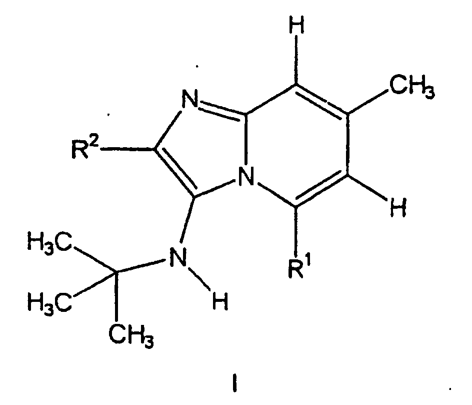 wherein
R¹ denotes H or a C₁₋₄ alkanyl, wherein the alkanyl is straight-chain or branched and is unsubstituted or singly or multiply substituted, and
R² denotes a C₁₋₈ alkyl, wherein the alkyl is straight-chain or branched, is saturated or unsaturated and is unsubstituted or singly or multiply substituted, a C₃₋₈ cycloalkyl, wherein the cycloalkyl is saturated or unsaturated and is unsubstituted or singly or multiply substituted, a heterocyclyl, wherein the heterocyclyl is saturated or unsaturated and is unsubstituted or singly or multiply substituted, an aryl, wherein the aryl is unsubstituted or singly or multiply substituted, a heteroaryl, wherein the heteroaryl is unsubstituted or singly or multiply substituted, a C₁₋₈ alkyl-C₃₋₈ cycloalkyl, a C₁₋₈ alkyl-heterocyclyl, a C₁₋₈ alkyl-aryl or a C₁₋₈ alkyl-heteroaryl, wherein the alkyl is straight-chain or branched, is saturated or unsaturated and is unsubstituted or singly or multiply substituted, the cycloalkyl is saturated or unsaturated and is unsubstituted or singly or multiply substituted, the heterocyclyl is saturated or unsaturated and is unsubstituted or singly or multiply substituted, the aryl is unsubstituted or singly or multiply substituted, and the heteroaryl is unsubstituted or singly or multiply substituted,
in the form of its base or of a pharmaceutically acceptable salt,
and which also contains at least one pharmaceutical auxiliary substance.

18. Use of a product for the preparation of an agent for the inhibition of NO synthase and/or for the treatment of migraine and/or for the treatment of septic shock, multiple sclerosis, Parkinson's medical condition, Alzheimer's medical condition, Huntington's medical condition, inflammation, pain due to inflammation, cerebral ischaemia, diabetes, meningitis, arteriosclerosis and/or for the healing of wounds with a compound of general structure I or of a pharmaceutically acceptable salt thereof 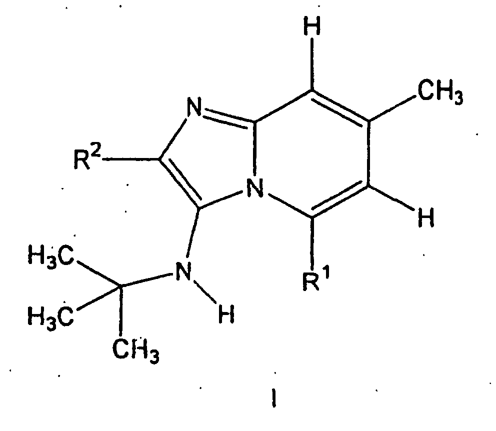 wherein
R¹ denotes H or a C₁₋₄ alkanyl, wherein the alkanyl is straight-chain or branched and is unsubstituted or singly or multiply substituted, and
R² denotes a C₁₋₈ alkyl, wherein the alkyl is straight-chain or branched, is saturated or unsaturated and is unsubstituted or singly or multiply substituted, a C₃₋₈ cycloalkyl, wherein the cycloalkyl is saturated or unsaturated and is unsubstituted or singly or multiply substituted, a heterocyclyl, wherein the heterocyclyl is saturated or unsaturated and is unsubstituted or singly or multiply substituted, an aryl, wherein the aryl is unsubstituted or singly or multiply substituted, a heteroaryl, wherein the heteroaryl is unsubstituted or singly or multiply substituted, a C₁₋₈ alkyl-C₃₋₈ cycloalkyl, a C₁₋₈ alkyl-heterocyclyl, a C₁₋₈ alkyl-aryl or a C₁₋₈ alkyl-heteroaryl, wherein the alkyl is straight-chain or branched, is saturated or unsaturated and is unsubstituted or singly or multiply substituted, the cycloalkyl is saturated or unsaturated and is unsubstituted or singly or multiply substituted, the heterocyclyl is saturated or unsaturated and is unsubstituted or singly or multiply substituted, the aryl is unsubstituted or singly or multiply substituted, and the heteroaryl is unsubstituted or singly or multiply substituted.

19. Use of a product for the preparation of an agent for the inhibition of NO synthase and/or for the treatment of migraine and/or for the treatment of septic shock, multiple sclerosis, Parkinson's medical condition, Alzheimer's medical condition, Huntington's medical condition, inflammation, pain due to inflammation, cerebral ischaemia, diabetes, meningitis, arteriosclerosis and/or for the healing of wounds in/on a mammal, particularly a human, **characterised in that** an effective amount of a compound of general structure I or of a pharmaceutically acceptable salt thereof 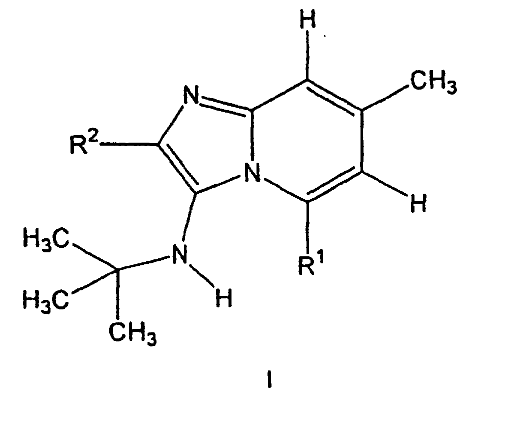 wherein
R¹ denotes H or a C₁₋₄ alkanyl, wherein the alkanyl is straight-chain or branched and is unsubstituted or singly or multiply substituted, and
R² denotes a C₁₋₈ alkyl, wherein the alkyl is straight-chain or branched, is saturated or unsaturated and is unsubstituted or singly or multiply substituted, a C₃₋₈ cycloalkyl, wherein the cycloalkyl is saturated or unsaturated and is unsubstituted or singly or multiply substituted, a heterocyclyl, wherein the heterocyclyl is saturated or unsaturated and is unsubstituted or singly or multiply substituted, an aryl, wherein the aryl is unsubstituted or singly or multiply substituted, a heteroaryl, wherein the heteroaryl is unsubstituted or singly or multiply substituted, a C₁₋₈ alkyl-C₃₋₈ cycloalkyl, a C₁₋₈ alkyl-heterocyclyl, a C₁₋₈ alkyl-aryl or a C₁₋₈ alkyl-heteroaryl, wherein the alkyl is straight-chain or branched, is saturated or unsaturated and is unsubstituted or singly or multiply substituted, the cycloalkyl is saturated or unsaturated and is unsubstituted or singly or multiply substituted, the heterocyclyl is saturated or unsaturated and is unsubstituted or singly or multiply substituted, the aryl is unsubstituted or singly or multiply substituted, and the heteroaryl is unsubstituted or singly or multiply substituted,
is used.

## Revendications

1. Composé de formule générale I 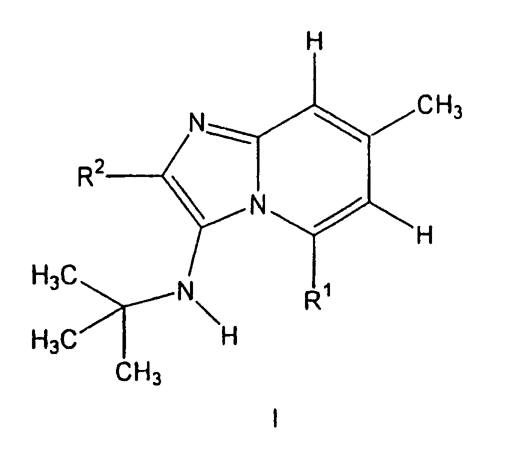 dans laquelle
R¹ représente H ou un reste alcanyle en C₁ à C₄, ce reste alcanyle étant linéaire ou ramifié et non substitué ou porteur d'un ou plusieurs substituants, et
R² est un reste alkyle en C₁ à C₈, ce reste alkyle étant linéaire ou ramifié et saturé ou non saturé et non substitué ou porteur d'un ou plusieurs substituants, un reste cycloalkyle en C₃ à C₈, ce reste cycloalkyle étant saturé ou non saturé et non substitué ou porteur d'un ou plusieurs substituants, un reste hétérocyclyle, ce reste hétérocyclyle étant saturé ou non saturé et non substitué ou porteur d'un ou plusieurs substituants, un reste aryle, ce reste aryle étant non substitué et porteur d'un ou plusieurs substituants, un reste hétéroaryle, ce reste hétéroaryle étant non substitué ou porteur d'un ou plusieurs substituants, un reste (alkyle en C₁ à C₈)-(cycloalkyle en C₃ à C₈), (alkyle en C₁ à C₈)-hétérocyclyle, (alkyle en C₁ à C₈)-aryle ou (alkyle en C₁ à C₈)-hétéroaryle, le radical alkyle étant linéaire ou ramifié et saturé ou non saturé et non substitué ou porteur d'un ou plusieurs substituants, le radical cycloalkyle étant saturé ou non saturé et non substitué ou porteur d'un ou plusieurs substituants, le radical hétérocyclyle étant saturé ou non saturé et non substitué ou porteur d'un ou plusieurs substituants, le radical aryle étant non substitué ou porteur d'un ou plusieurs substituants et le radical hétéroaryle étant non substitué ou porteur d'un ou plusieurs substituants,
sous forme de sa base ou d'un sel pharmaceutiquement acceptable, à l'exception du composé de formule générale I dans laquelle R¹ est un groupe méthyle et R² est un groupe phényle.

2. Composé suivant la revendication 1, dans lequel R¹ représente H ou le groupe méthyle, sous forme de sa base ou d'un sel pharmaceutiquement acceptable.

3. Composé suivant la revendication 1 ou 2, dans lequel R² est un reste aryle ou hétéroaryle, sous forme de sa base ou d'un sel pharmaceutiquement acceptable.

4. Composé suivant la revendication 3, dans lequel R² est un reste 1-naphtyle, phényle, furyle, thiényle ou pyridinyle et est non substitué ou porte un ou plusieurs substituants, sous forme de sa base ou d'un sel pharmaceutiquement acceptable.

5. Composé suivant la revendication 4, dans lequel le reste 1-naphtyle, phényle, furyle, thiényle ou pyridinyle est non substitué ou porte un ou deux substituants -F, -CF₃, -OH, -O-CH₃, -CH₂OH, -NO₂, -CO₂H et/ou-[1,3]-dioxolane, sous forme de sa base ou d'un sel pharmaceutiquement acceptable.

6. Composé suivant l'une des revendications 1 à 5 sous forme de sa base ou d'un sel pharmaceutiquement acceptable, en particulier sous forme de son chlorhydrate, choisi entre les composés suivants :
tertiobutyl-(7-méthyl-2-pyridine-3-yl-imidazo[1,2-a]-pyridine-3-yl)amine,
tertiobutyl-[2-(2,3-diméthoxyphényl)-5,7-diméthyl-imidazo-[1,2-a]pyridine-3-yl]amine,
tertiobutyl-(2,5,7-triméthyl-imidazo[1,2-a]pyridine-3-yl)-amine,
3-(3-tertiobutylamino-5,7-diméthyl-imidazo[1,2-a]pyridine-2-yl)phénol,
tertiobutyl-(2-cyclohexyl-5,7-diméthyl-imidazo[1,2-a]-pyridine-3-yl)amine,
3-(3-tertiobutylamino-7-méthyl-imidazo[1,2-a]pyridine-2-yl)phénol,
tertiobutyl-(2-furanne-2-yl-5,7-diméthylimidazo[1,2-a]-pyridine-3-yl)amine,
tertiobutyl-(7-méthyl-2-naphtalène-1-yl-imidazo[1,2-a]-pyridine-3-yl)amine,
tertiobutyl-[5,7-diméthyl-2-(5-nitro-furanne-2-yl)-imidazo-[1,2-a]pyridine-3-yl]amine,
[5-(3-tertiobutylamino-7-méthyl-imidazo[1,2-a]pyridine-2-yl)-furanne-2-yl]méthanol,
tertiobutyl-[2-(5-[1,3]dioxolane-2-yl-furanne-2-yl)-7-méthyl-imidazo[1,2-a]pyridine-3-yl)amine,
acide 5-(3-tertiobutylamino-5,7-diméthyl-imidazo[1,2-a]-pyridine-2-yl)-furanne-2-carboxylique,
tertiobutyl-(2-furanne-2-yl-7-méthyl-imidazo[1,2-a]-pyridine-3-yl)amine,
tertiobutyl-(7-méthyl-2-pyridine-2-yl-imidazo[1,2-a]-pyridine-3-yl)amine,
tertiobutyl-(2-cyclohexyl-7-méthyl-imidazo[1,2-a]pyridine-3-yl)amine,
tertiobutyl-(2,7-diméthyl-imidazo[1,2-a]pyridine-3-yl)-amine,
acide 5-(3-tertiobutylamino-7-méthyl-imidazo[1,2-a]-pyridine-2-yl)-thiophène-2-carboxylique,
tertiobutyl-(7-méthyl-2-phényl-imidazo[1,2-a]pyridine-3-yl)amine.

7. Procédé de production d'un composé de formule générale I suivant l'une des revendications 1 à 6 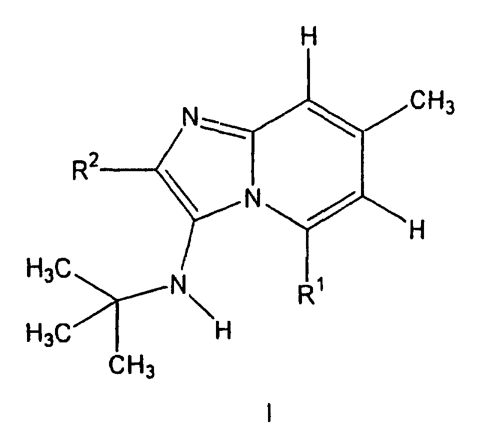 **caractérisé en ce que** l'on fait réagir une aminopyridine de formule générale II 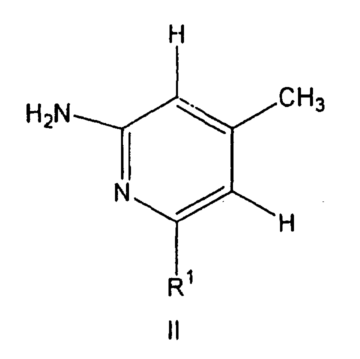 dans laquelle
R¹ représente H ou un reste alcanyle en C₁ à C₄, ce reste alcanyle étant linéaire ou ramifié et non substitué ou porteur d'un ou plusieurs substituants.
avec un aldéhyde de formule générale III 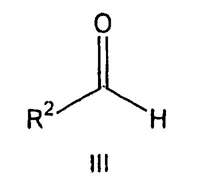 dans laquelle
R² est un reste alkyle en C₁ à C₈, ce reste alkyle étant linéaire ou ramifié et saturé ou non saturé et étant non substitué ou porteur d'un ou plusieurs substituants, un reste cycloalkyle en C₃ à C₈, ce reste cycloalkyle étant saturé ou non saturé et étant non substitué ou porteur d'un ou plusieurs substituants, un reste hétérocyclyle, ce reste hétérocyclyle étant saturé ou non saturé et étant non substitué ou porteur d'un ou plusieurs substituants, un reste aryle, ce reste aryle étant non substitué ou porteur d'un ou plusieurs substituants, un reste hétéroaryle, ce reste hétéroaryle étant non substitué ou porteur d'un ou plusieurs substituants, un reste (alkyle en C₁ à C₈)-(cycloalkyle en C₃ à C₈), (alkyle en C₁ à C₈)-hétérocyclyle, (alkyle en C₁ à C₈)-aryle ou (alkyle en C₁ à C₈)-hétéroaryle, le radical alkyle étant linéaire ou ramifié et saturé ou non saturé et étant non substitué ou porteur d'un ou plusieurs substituants, le radical cycloalkyle étant saturé ou non saturé et étant non substitué ou porteur d'un ou plusieurs substituants, le radical hétérocyclyle étant saturé ou non saturé et étant non substitué ou porteur d'un ou plusieurs substituants, le radical aryle étant non substitué ou porteur d'un ou plusieurs substituants et le radical hétéroaryle étant non substitué ou porteur d'un ou plusieurs substituants, sous réserve que R² ne représente pas un groupe phényle lorsque R¹ représente un groupe méthyle,
et le tertiobutylisonitrile de formule IV

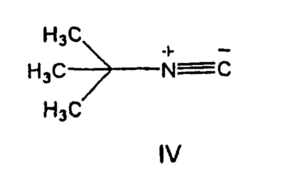

8. Procédé suivant la revendication 7, **caractérisé en ce qu'**il est mis en oeuvre en présence d'un acide, en particulier l'acide perchlorique.

9. Procédé suivant l'une des revendications 7 et 8, **caractérisé en ce que** la réaction de l'aminopyridine II avec l'aldéhyde III et le tertiobutylisonitrile IV est conduite selon un procédé en récipient unique.

10. Procédé suivant l'une des revendications 7 à 9, **caractérisé en ce que** la base formée de formule générale I est transformée en son chlorhydrate par addition de triméthylchlorosilane.

11. Médicament contenant au moins un composé de formule générale I 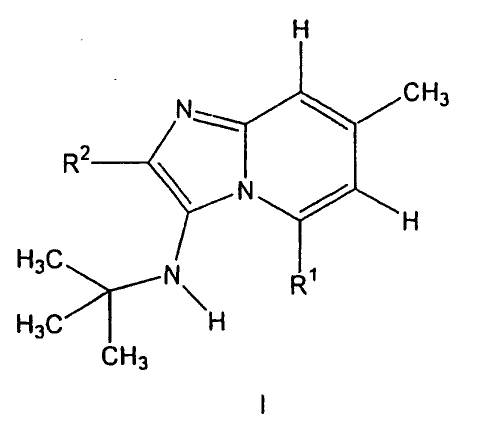 dans laquelle
R¹ représente H ou un reste alcanyle en C₁ à C₄, ce reste alcanyle étant linéaire ou ramifié et non substitué ou porteur d'un ou plusieurs substituants, et
R² est un reste alkyle en C₁ à C₈, ce reste alkyle étant linéaire ou ramifié et saturé ou non saturé et non substitué ou porteur d'un ou plusieurs substituants, un reste cycloalkyle en C₃ à C₈, ce reste cycloalkyle étant saturé ou non saturé et non substitué ou porteur d'un ou plusieurs substituants, un reste hétérocyclyle, ce reste hétérocyclyle étant saturé ou non saturé et non substitué ou porteur d'un ou plusieurs substituants, un reste aryle, ce reste aryle étant non substitué et porteur d'un ou plusieurs substituants, un reste hétéroaryle, ce reste hétéroaryle étant non substitué ou porteur d'un ou plusieurs substituants, un reste (alkyle en C₁ à C₈)-(cycloalkyle en C₃ à C₈), (alkyle en C₁ à C₈)-hétérocyclyle, (alkyle en C₁ à C₈)-aryle ou (alkyle en C₁ à C₈)-hétéroaryle, le radical alkyle étant linéaire ou ramifié et saturé ou non saturé et non substitué ou porteur d'un ou plusieurs substituants, le radical cycloalkyle étant saturé ou non saturé et non substitué ou porteur d'un ou plusieurs substituants, le radical hétérocyclyle étant saturé ou non saturé et non substitué ou porteur d'un ou plusieurs substituants, le radical aryle étant non substitué ou porteur d'un ou plusieurs substituants et le radical hétéroaryle étant non substitué ou porteur d'un ou plusieurs substituants,
sous forme de sa base ou d'un sel pharmaceutiquement acceptable.

12. Utilisation d'un composé de formule générale I 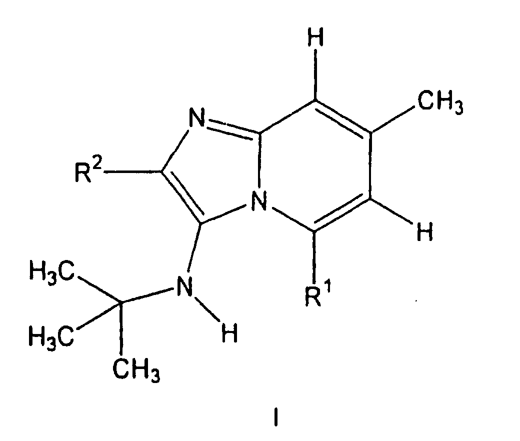 dans laquelle
R¹ représente H ou un reste alcanyle en C₁ à C₄, ce reste alcanyle étant linéaire ou ramifié et non substitué ou porteur d'un ou plusieurs substituants, et
R² est un reste alkyle en C₁ à C₈, ce reste alkyle étant linéaire ou ramifié et saturé ou non saturé et non substitué ou porteur d'un ou plusieurs substituants, un reste cycloalkyle en C₃ à C₈, ce reste cycloalkyle étant saturé ou non saturé et non substitué ou porteur d'un ou plusieurs substituants, un reste hétérocyclyle, ce reste hétérocyclyle étant saturé ou non saturé et non substitué ou porteur d'un ou plusieurs substituants, un reste aryle, ce reste aryle étant non substitué et porteur d'un ou plusieurs substituants, un reste hétéroaryle, ce reste hétéroaryle étant non substitué ou porteur d'un ou plusieurs substituants, un reste (alkyle en C₁ à C₈)-(cycloalkyle en C₃ à C₈), (alkyle en C₁ à C₈)-hétérocyclyle, (alkyle en C₁ à C₈)-aryle ou (alkyle en C₁ à C₈)-hétéroaryle, le radical alkyle étant linéaire ou ramifié et saturé ou non saturé et non substitué ou porteur d'un ou plusieurs substituants, le radical cycloalkyle étant saturé ou non saturé et non substitué ou porteur d'un ou plusieurs substituants, le radical hétérocyclyle étant saturé ou non saturé et non substitué ou porteur d'un ou plusieurs substituants, le radical aryle étant non substitué ou porteur d'un ou plusieurs substituants et le radical hétéroaryle étant non substitué ou porteur d'un ou plusieurs substituants,
sous forme de sa base ou d'un sel pharmaceutiquement acceptable,
pour la préparation d'un médicament destiné à inhiber la NO-synthase.

13. Utilisation suivant la revendication 12, **caractérisée en ce que** le composé de formule générale I est choisi parmi les composés suivants :
tertiobutyl-(7-méthyl-2-pyridine-3-yl-imidazo[1,2-a]-pyridine-3-yl)amine,
tertiobutyl-[2-(2,3-diméthoxyphényl)-5,7-diméthyl-imidazo-[1,2-a]pyridine-3-yl]amine,
tertiobutyl-(2,5,7-triméthyl-imidazo[1,2-a]pyridine-3-yl)-amine,
3-(3-tertiobutylamino-5,7-diméthyl-imidazo[1,2-a]pyridine-2-yl)phénol,
tertiobutyl-(2-cyclohexyl-5,7-diméthyl-imidazo[1,2-a]-pyridine-3-yl)amine,
3-(3-tertiobutylamino-7-méthyl-imidazo[1,2-a]pyridine-2-yl)phénol,
tertiobutyl-(2-furanne-2-yl-5,7-diméthylimidazo[1,2-a]-pyridine-3-yl)amine,
tertiobutyl-(7-méthyl-2-naphtalène-1-yl-imidazo[1,2-a]-pyridine-3-yl)amine,
tertiobutyl-[5,7-diméthyl-2-(5-nitro-furanne-2-yl)-imidazo-[1,2-a]pyridine-3-yl]amine,
[5-(3-tertiobutylamino-7-méthyl-imidazo[1,2-a]pyridine-2-yl)-furanne-2-yl]méthanol,
tertiobutyl-[2-(5-[1,3]dioxolane-2-yl-furanne-2-yl)-7-méthyl-imidazo[1,2-a]pyridine-3-yl)amine,
acide 5-(3-tertiobutylamino-5,7-diméthyl-imidazo[1,2-a]-pyridine-2-yl)-furanne-2-carboxylique,
tertiobutyl-(2-furanne-2-yl-7-méthyl-imidazo[1,2-a]-pyridine-3-yl)amine,
tertiobutyl-(7-méthyl-2-pyridine-2-yl-imidazo[1,2-a]-pyridine-3-yl)amine,
tertiobutyl-(2-cyclohexyl-7-méthyl-imidazo[1,2-a]pyridine-3-yl)amine,
tertiobutyl-(2,7-diméthyl-imidazo[1,2-a]pyridine-3-yl)-amine,
acide 5-(3-tertiobutylamino-7-méthyl-imidazo[1,2-a]-pyridine-2-yl)-thiophène-2-carboxylique,
tertiobutyl-(7-méthyl-2-phényl-imidazo[1,2-a]pyridine-3-yl)amine.

14. Utilisation d'un composé de formule générale I 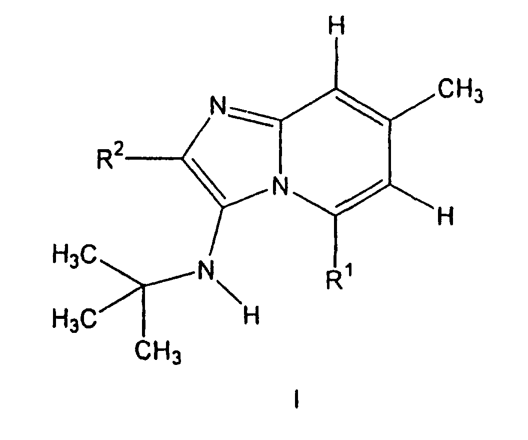 dans laquelle
R¹ représente H ou un reste alcanyle en C₁ à C₄, ce reste alcanyle étant linéaire ou ramifié et non substitué ou porteur d'un ou plusieurs substituants, et
R² est un reste alkyle en C₁ à C₈, ce reste alkyle étant linéaire ou ramifié et saturé ou non saturé et non substitué ou porteur d'un ou plusieurs substituants, un reste cycloalkyle en C₃ à C₈, ce reste cycloalkyle étant saturé ou non saturé et non substitué ou porteur d'un ou plusieurs substituants, un reste hétérocyclyle, ce reste hétérocyclyle étant saturé ou non saturé et non substitué ou porteur d'un ou plusieurs substituants, un reste aryle, ce reste aryle étant non substitué ou porteur d'un ou plusieurs substituants, un reste hétéroaryle, ce reste hétéroaryle étant non substitué ou porteur d'un ou plusieurs substituants, un reste (alkyle en C₁ à C₈)-(cycloalkyle en C₃ à C₈), (alkyle en C₁ à C₈)-hétérocyclyle, (alkyle en C₁ à C₈)-aryle ou (alkyle en C₁ à C₈)-hétéroaryle, le radical alkyle étant linéaire ou ramifié et saturé ou non saturé et non substitué ou porteur d'un ou plusieurs substituants, le radical cycloalkyle étant saturé ou non saturé et non substitué ou porteur d'un ou plusieurs substituants, le radical hétérocyclyle étant saturé ou non saturé et non substitué ou porteur d'un ou plusieurs substituants, le radical aryle étant non substitué ou porteur d'un ou plusieurs substituants et le radical hétéroaryle étant non substitué ou porteur d'un ou plusieurs substituants,
sous forme de sa base ou d'un sel pharmaceutiquement acceptable, pour la préparation d'un médicament destiné au traitement de la migraine.

15. Utilisation suivant la revendication 14, **caractérisée en ce que** le composé de formule générale I est choisi entre les composés suivants :
tertiobutyl-(7-méthyl-2-pyridine-3-yl-imidazo[1,2-a]-pyridine-3-yl)amine,
tertiobutyl-[2-(2,3-diméthoxyphényl)-5,7-diméthyl-imidazo-[1,2-a]pyridine-3-yl]amine,
tertiobutyl-(2,5,7-triméthyl-imidazo[1,2-a]pyridine-3-yl)-amine,
3-(3-tertiobutylamino-5,7-diméthyl-imidazo[1,2-a]pyridine-2-yl)phénol,
tertiobutyl-(2-cyclohexyl-5,7-diméthyl-imidazo[1,2-a]-pyridine-3-yl)amine,
3-(3-tertiobutylamino-7-méthyl-imidazo[1,2-a]pyridine-2-yl)phénol,
tertiobutyl-(2-furanne-2-yl-5,7-diméthylimidazo[1,2-a]-pyridine-3-yl)amine,
tertiobutyl-(7-méthyl-2-naphtalène-1-yl-imidazo[1,2-a]-pyridine-3-yl)amine,
tertiobutyl-[5,7-diméthyl-2-(5-nitro-furanne-2-yl)-imidazo-[1,2-a]pyridine-3-yl]amine,
[5-(3-tertiobutylamino-7-méthyl-imidazo[1,2-a]pyridine-2-yl)-furanne-2-yl]méthanol,
tertiobutyl-[2-(5-[1,3]dioxolane-2-yl-furanne-2-yl)-7-méthyl-imidazo [1,2-a]pyridine-3-yl)amine,
acide 5-(3-tertiobutylamino-5,7-diméthyl-imidazo[1,2-a]-pyridine-2-yl)-furanne-2-carboxylique,
tertiobutyl-(2-furanne-2-yl-7-méthyl-imidazo[1,2-a]-pyridine-3-yl)amine,
tertiobutyl-(7-méthyl-2-pyridine-2-yl-imidazo[1,2-a]-pyridine-3-yl)amine,
tertiobutyl-(2-cyclohexyl-7-méthyl-imidazo[1,2-a]pyridine-3-yl)amine,
tertiobutyl-(2,7-diméthyl-imidazo[1,2-a]pyridine-3-yl)-amine,
acide 5-(3-tertiobutylamino-7-méthyl-imidazo[1,2-a]-pyridine-2-yl)-thiophène-2-carboxylique,
tertiobutyl-(7-méthyl-2-phényl-imidazo[1,2-a]pyridine-3-yl)amine.

16. Utilisation d'un composé de formule générale I 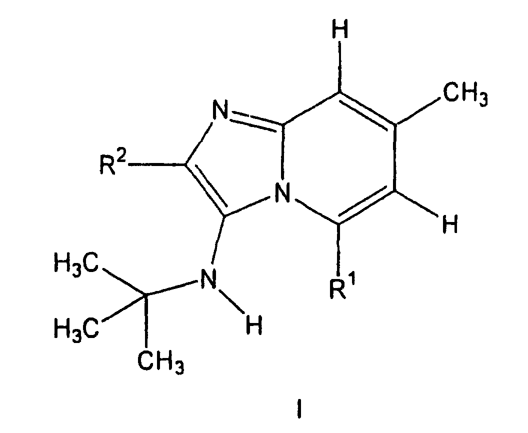 dans laquelle
R¹ représente H ou un reste alcanyle en C₁ à C₄, ce reste alcanyle étant linéaire ou ramifié et non substitué ou porteur d'un ou plusieurs substituants, et
R² est un reste alkyle en C₁ à C₈, ce reste alkyle étant linéaire ou ramifié et saturé ou non saturé et non substitué ou porteur d'un ou plusieurs substituants, un reste cycloalkyle en C₃ à C₈, ce reste cycloalkyle étant saturé ou non saturé et non substitué ou porteur d'un ou plusieurs substituants, un reste hétérocyclyle, ce reste hétérocyclyle étant saturé ou non saturé et non substitué ou porteur d'un ou plusieurs substituants, un reste aryle, ce reste aryle étant non substitué et porteur d'un ou plusieurs substituants, un reste hétéroaryle, ce reste hétéroaryle étant non substitué ou porteur d'un ou plusieurs substituants, un reste (alkyle en C₁ à C₈)-(cycloalkyle en C₃ à C₈), (alkyle en C₁ à C₈)-hétérocyclyle, (alkyle en C₁ à C₈)-aryle ou (alkyle en C₁ à C₈)-hétéroaryle, le radical alkyle étant linéaire ou ramifié et saturé ou non saturé et non substitué ou porteur d'un ou plusieurs substituants, le radical cycloalkyle étant saturé ou non saturé et non substitué ou porteur d'un ou plusieurs substituants, le radical hétérocyclyle étant saturé ou non saturé et non substitué ou porteur d'un ou plusieurs substituants, le radical aryle étant non substitué ou porteur d'un ou plusieurs substituants et le radical hétéroaryle étant non substitué ou porteur d'un ou plusieurs substituants,
sous forme de sa base ou d'un sel pharmaceutiquement acceptable, pour la préparation d'un médicament destiné au traitement du choc septique, de la sclérose en plaques, de la maladie de Parkinson, de la maladie d'Alzheimer, de la maladie d'Huntington, d'inflammations, d'une douleur inflammatoire, de l'ischémie cérébrale, du diabète, de la méningite, de l'artériosclérose et/ou pour la cicatrisation.

17. Composition pharmaceutique, qui contient au moins un composé de formule générale I 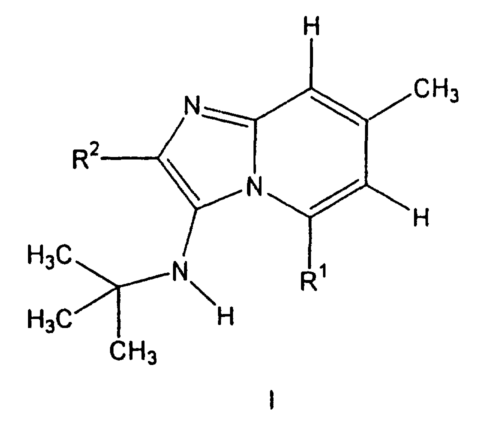 dans laquelle
R¹ représente H ou un reste alcanyle en C₁ à C₄, ce reste alcanyle étant linéaire ou ramifié et non substitué ou porteur d'un ou plusieurs substituants, et
R² est un reste alkyle en C₁ à C₈, ce reste alkyle étant linéaire ou ramifié et saturé ou non saturé et non substitué ou porteur d'un ou plusieurs substituants, un reste cycloalkyle en C₃ à C₈, ce reste cycloalkyle étant saturé ou non saturé et non substitué ou porteur d'un ou plusieurs substituants, un reste hétérocyclyle, ce reste hétérocyclyle étant saturé ou non saturé et non substitué ou porteur d'un ou plusieurs substituants, un reste aryle, ce reste aryle étant non substitué et porteur d'un ou plusieurs substituants, un reste hétéroaryle, ce reste hétéroaryle étant non substitué ou porteur d'un ou plusieurs substituants, un reste (alkyle en C₁ à C₈)-(cycloalkyle en C₃ à C₈), (alkyle en C₁ à C₈)-hétérocyclyle, (alkyle en C₁ à C₈)-aryle ou (alkyle en C₁ à C₈)-hétéroaryle, le radical alkyle étant linéaire ou ramifié et saturé ou non saturé et non substitué ou porteur d'un ou plusieurs substituants, le radical cycloalkyle étant saturé ou non saturé et non substitué ou porteur d'un ou plusieurs substituants, le radical hétérocyclyle étant saturé ou non saturé et non substitué ou porteur d'un ou plusieurs substituants, le radical aryle étant non substitué ou porteur d'un ou plusieurs substituants et le radical hétéroaryle étant non substitué ou porteur d'un ou plusieurs substituants,
sous forme de sa base ou d'un sel pharmaceutiquement acceptable, ainsi qu'au moins une substance pharmaceutique auxiliaire.

18. Utilisation d'un produit pour la préparation d'un agent destiné à l'inhibition de la NO-synthase et/ou au traitement de la migraine et/ou au traitement du choc septique, de la sclérose en plaques, de la maladie de Parkinson, de la maladie d'Alzheimer, de la maladie d'Huntington, d'inflammations, d'une douleur inflammatoire, de l'ischémie cérébrale, du diabète, de la méningite, de l'artériosclérose et/ou pour la cicatrisation, avec un composé de formule générale I ou un sel pharmaceutiquement acceptable de ce composé, 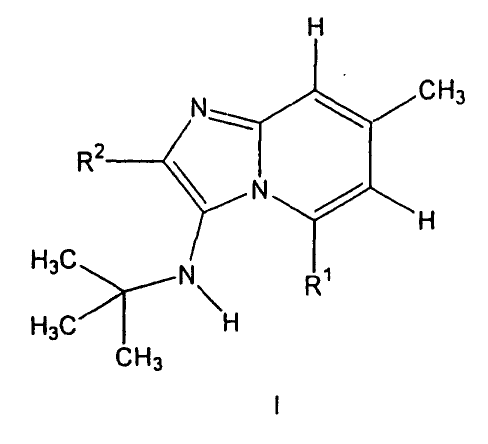 formule dans laquelle
R¹ représente H ou un reste alcanyle en C₁ à C₄, ce reste alcanyle étant linéaire ou ramifié et non substitué ou porteur d'un ou plusieurs substituants, et
R² est un reste alkyle en C₁ à C₈, ce reste alkyle étant linéaire ou ramifié et saturé ou non saturé et non substitué ou porteur d'un ou plusieurs substituants, un reste cycloalkyle en C₃ à C₈, ce reste cycloalkyle étant saturé ou non saturé et non substitué ou porteur d'un ou plusieurs substituants, un reste hétérocyclyle, ce reste hétérocyclyle étant saturé ou non saturé et non substitué ou porteur d'un ou plusieurs substituants, un reste aryle, ce reste aryle étant non substitué et porteur d'un ou plusieurs substituants, un reste hétéroaryle, ce reste hétéroaryle étant non substitué ou porteur d'un ou plusieurs substituants, un reste (alkyle en C₁ à C₈)-(cycloalkyle en C₃ à C₈), (alkyle en C₁ à C₈)-hétérocyclyle, (alkyle en C₁ à C₈)-aryle ou (alkyle en C₁ à C₈)-hétéroaryle, le radical alkyle étant linéaire ou ramifié et saturé ou non saturé et non substitué ou porteur d'un ou plusieurs substituants, le radical cycloalkyle étant saturé ou non saturé et non substitué ou porteur d'un ou plusieurs substituants, le radical hétérocyclyle étant saturé ou non saturé et non substitué ou porteur d'un ou plusieurs substituants, le radical aryle étant non substitué ou porteur d'un ou plusieurs substituants et le radical hétéroaryle étant non substitué ou porteur d'un ou plusieurs substituants.

19. Utilisation d'un produit pour la préparation d'un agent destiné à l'inhibition de la NO-synthase et/ou au traitement de la migraine et/ou au traitement du choc septique, de la sclérose en plaques, de la maladie de Parkinson, de la maladie d'Alzheimer, de la maladie d'Huntington, d'inflammations, d'une douleur inflammatoire, de l'ischémie cérébrale, du diabète, de la méningite, de l'artériosclérose et/ou pour la cicatrisation dans/sur un mammifère, en particulier un être humain, **caractérisée en ce que** l'on utilise un composé de formule générale I ou un sel pharmaceutiquement acceptable de ce composé 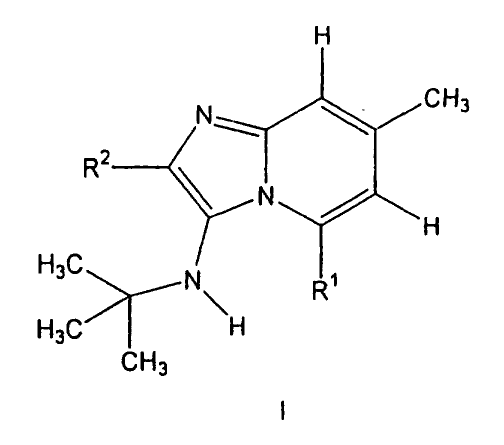 formule dans laquelle
R¹ représente H ou un reste alcanyle en C₁ à C₄, ce reste alcanyle étant linéaire ou ramifié et non substitué ou porteur d'un ou plusieurs substituants, et
R² est un reste alkyle en C₁ à C₈, ce reste alkyle étant linéaire ou ramifié et saturé ou non saturé et non substitué ou porteur d'un ou plusieurs substituants, un reste cycloalkyle en C₃ à C₈, ce reste cycloalkyle étant saturé ou non saturé et non substitué ou porteur d'un ou plusieurs substituants, un reste hétérocyclyle, ce reste hétérocyclyle étant saturé ou non saturé et non substitué ou porteur d'un ou plusieurs substituants, un reste aryle, ce reste aryle étant non substitué et porteur d'un ou plusieurs substituants, un reste hétéroaryle, ce reste hétéroaryle étant non substitué ou porteur d'un ou plusieurs substituants, un reste (alkyle en C₁ à C₈)-(cycloalkyle en C₃ à C₈), (alkyle en C₁ à C₈)-hétérocyclyle, (alkyle en C₁ à C₈)-aryle ou (alkyle en C₁ à C₈)-hétéroaryle, le radical alkyle étant linéaire ou ramifié et saturé ou non saturé et non substitué ou porteur d'un ou plusieurs substituants, le radical cycloalkyle étant saturé ou non saturé et non substitué ou porteur d'un ou plusieurs substituants, le radical hétérocyclyle étant saturé ou non saturé et non substitué ou porteur d'un ou plusieurs substituants, le radical aryle étant non substitué ou porteur d'un ou plusieurs substituants et le radical hétéroaryle étant non substitué ou porteur d'un ou plusieurs substituants.
